(19) Europäisches Patentamt
European Patent Office
Office européen des brevets

(11) Veröffentlichungsnummer: **0 131 226**
**B1**

(12) **EUROPÄISCHE PATENTSCHRIFT**

(45) Veröffentlichungstag der Patentschrift: **30.05.90**

(51) Int. Cl.$^5$: **C 07 D 209/52,** A 61 K 31/40

(21) Anmeldenummer: **84107607.8**

(22) Anmeldetag: **30.06.84**

(54) **Derivate der 2-Azabicyclo(3.1.0)hexan-3-carbonsäure, Verfahren zu ihrer Herstellung, diese enthaltende Mittel und deren Verwendung sowie 2-Azabicyclo(3.1.0)hexan-Derivate als Zwischenprodukte und Verfahren zu deren Herstellung.**

(30) Priorität: **06.07.83 DE 3324263**

(43) Veröffentlichungstag der Anmeldung:
**16.01.85 Patentblatt 85/03**

(45) Bekanntmachung des Hinweises auf die Patenterteilung:
**30.05.90 Patentblatt 90/22**

(84) Benannte Vertragsstaaten:
**AT BE CH DE FR GB IT LI LU NL SE**

(56) Entgegenhaltungen:
**EP-A-0 051 301**
**EP-A-0 079 022**
**EP-A-0 084 164**

(73) Patentinhaber: **HOECHST AKTIENGESELLSCHAFT**
**Postfach 80 03 20**
**D-6230 Frankfurt am Main 80 (DE)**

(72) Erfinder: **Urbach, Hansjörg, Dr.**
**Le Lavandoustrasse 41**
**D-6242 Kronberg/Taunus (DE)**
Erfinder: **Henning, Rainer, Dr.**
**Rotenhofstrasse 31**
**D-6234 Hattersheim am Main (DE)**
Erfinder: **Becker, Reinhard, Dr.**
**Adelheidstrasse 101**
**D-6200 Wiesbaden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 131 226 B1

**Beschreibung**

Aus der EP—A2—0079022 sind cis, endo-2-Azabicyclo[3,3,0]octan-3-carbonsäure-Derivate, aus der EP—A1—0051301 Indolin-2-carbonsäure-Derivate bekannt. In den Dokumenten werden außerdem Verfahren zur Herstellung der genannten Verbindungen und deren Verwendugn als Hemmer des Angiotensin-Converting Enzyms (ACE) beschrieben.

Es wurden neue 2-Azabicyclo[3,1,0]hexan-2-carbonsäure-Derivate gefunden, die hochwirksam das ACE hemmen.

Die Erfindung betrifft daher Verbindungen der Formel I

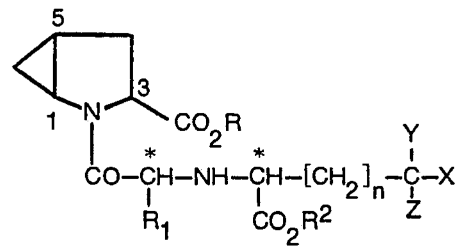

(I)

in der die Wasserstoffatome an den Brückenkopf-C-Atomen zueinander cis-konfiguriert sind und die COOR-Gruppe am C-Atom 3 exo- oder endoständig zum bicyclischen Ringsystem orientiert ist und worin n = 0 oder 1, R = Wasserstoff, ($C_1$—$C_6$)-Alkyl, ($C_2$—$C_6$)-Alkenyl oder ($C_6$—$C_{12}$)-Aryl-($C_1$—$C_4$)-alkyl, $R^1$ = Wasserstoff oder ($C_1$—$C_6$)-Alkyl, das gegebenenfalls durch Amino, ($C_1$—$C_6$)-Acylamino oder Benzoylamino substituiert sein kann, ($C_2$—$C_6$)-Alkenyl, ($C_5$—$C_9$)-Cycloalkyl, ($C_5$—$C_9$)-Cycloalkenyl, ($C_5$—$C_7$)-Cycloalkyl-($C_1$—$C_4$)-alkyl, ($C_6$—$C_{12}$)-Aryl oder teilhydriertes ($C_6$—$C_{12}$)-Aryl, das jeweils durch ($C_1$—$C_4$)-Alkyl, ($C_1$ oder $C_2$)-Alkoxy oder Halogen substituiert sein kann, ($C_6$—$C_{12}$)-Aryl-($C_1$—$C_4$)-alkyl oder ($C_7$—$C_{13}$)-Aroyl-($C_1$—$C_2$) alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seiten kette einer natürlich vorkommenden α-Aminosäure $R^1$—CH($NH)_2$)-COOH,

$R^2$ = Wasserstoff, ($C_1$—$C_6$)-Alkyl, ($C_2$—$C_6$)-Alkenyl, oder ($C_6$—$C_{12}$)-Aryl-($C_1$—$C_4$)-alkyl,

Y = Wasserstoff oder Hydroxy,

Z = Wasserstoff oder

Y und Z = zusammen Sauerstoff,

X = ($C_1$—$C_6$-Alkyl, ($C_2$—$C_6$)-Alkenyl, ($C_5$-$C_9$)-Cycloalkyl, ($C_6$—$C_{12}$)-Aryl, vorzugsweise Phenyl, das durch ($C_1$—$C_4$)-Alkyl, ($C_1$—$C_4$)-Alkoxy, Hydroxy, Halogen, Nitro, Amino, ($C_1$—$C_4$)-Alkylamino, Di-($C_1$—$C_4$)-alkylamino und/oder Methylendioxy mono-, di- oder trisubstituiert sein kann, oder Indol-3-yl bedeuten, sowie deren physiologisch unbedenkliche Salze.

Bevorzugt sind Verbindungen der Formel I, in der

n = 1 ist,

$R^1$ = Wasserstoff, Allyl, Vinyl oder eine gegebenenfalls geschützte Seitenkette einer natürlich vorkommenden α-Aminosäure bedeutet und

R, $R^2$, Y, Z und X die vorstehenden Bedeutungen haben, insbesondere solche Verbindungen der Formel I, in der

n = 1,

R = Wasserstoff,

$R^1$ = Methyl, die gegebenenfalls acylierte Seitenkette von Lysin oder die O—($C_1$—$C_6$)-alkylierte Seitenkette des Tyrosin,

$R^2$ = Wasserstoff, Methyl, Ethyl, Benzyl oder tert. Butyl,

X = Phenyl oder mit Fluor und/oder Chlor mono- oder disubstituiertes Phenyl,

Y = Wasserstoff oder Hydroxy

Z = Wasserstoff oder

Y und Z zusammen Sauerstoff bedeuten.

Als besonders bevorzugte Verbindungen seien erwähnt:

N-(1-S-Carbethoxy-3-phenyl-propyl)-S-alanyl-cis-2-azabicyclo[3,1,0]hexan-endo-3-S-carbonsäure,

N-(1-S-Carboxy-3-phenyl-propyl)-S-alanyl-cis-2-azabicyclo-[3,1,0]hexan-endo-3-S-carbonsäure,

N-(1-S-Carbethoxy-3-phenyl-propyl)-S-lysyl-cis-2-azabicyclo[3,1,0]hexan-endo-3-S-carbonsäure,

N-(1-S-Carboxy-3-phenyl-propyl)-S-lysyl-cis-2-azabicyclo[3,1,0]hexan-endo-3-S-carbonsäure,

N-(1-S-Carbethoxy-3-phenyl-propyl)-O-ethyl-S-tyrosyl-cis-2-azabicyclo[3,1,0]hexan-endo-3-S-carbonsäure,

N-(1-S-Carbethoxy-3-phenyl-propyl)-O-methyl-S-tyrosyl-cis-2-azabicyclo[3,1,0]hexan-endo-3-S-carbonsäure,

N-(1-S-Carbethoxy-3-phenyl-propyl)-S-alanyl-cis-2-azabicyclo[3,1,0]hexane-exo-3-S-carbonsäure,

N-(1-S-Carboxy-3-phenyl-propyl)-S-alanyl-cis-2-azabicyclo[3,1,0]hexan-exo-3-S-carbonsäure,

N-(1-S-Carboxy-3-phenyl-propyl)-S-alanyl-cis-2-azabicyclo[3,1,0]hexan-exo-3-S-carbonsäure,
N-(1-S-Carbethoxy-3-phenyl-propyl)-S-lysyl-cis-2-azabicyclo[3,1,0]hexan-exo-3-S-carbonsäure,
N-(1-S-Carboxy-3-phenyl-propyl)-S-lysyl-cis-2-azabicyclo[3,1,0]hexan-endo-3-S-carbonsäure,
N-(1-S-Carbethoxy-3-phenyl-propyl)-O-ethyl-S-tyrosyl-cis-2-azabicyclo[3,1,0]hexan-exo-3-S-carbonsäure und
N-(1-S-Carbethoxy-3-phenyl-propyl)-O-methyl-S-tyrosyl-cis-2-azabicyclo[3,1,0]hexan-exo-3-S-carbonsäure.

Als Salze kommen insbesondere Alkali- und erdalkalisalze, Salze mit physiologisch verträglichen Aminen und Salze mit anorganischen oder organischen Säuren wie z.B. HCl, HBr, $H_2SO_4$, Maleinsäure, Furmarsäure oder Weinsäure in Frage.

Unter Aryl ist hier wie im folgenden gegebenenfalls substituiertes Phenyl, Naphthyl oder Biphenylyl, insbesondere aber Phenyl zu verstehen. Alkyl kann geradkettig oder verzweigt sein. Unter Acylamino wird insbesondere ($C_1$—$C_6$)-Alkanoylamino, Boc-Amino oder Benzoylamino verstanden.

Unter einem mono- bzw. bicyclischen Heterocylen-Rest mit 5 bis 7 bzw. 8 bis 10 Ringatomen, woven 1 bis 2 Ringatome, Schwefel- oder Sauerstoffatome und/oder wovon 1 bis 4 Ringatome Stickstoffatome darstellen, wird beispielsweise Thienyl, Benzo[b] thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Natürlich vorkommende α-Aminosäuren sind z.B. im Houben-Weyl, Bd. XV/1 und XV/2 beschrieben.

Falls $R^1$ für eine geschützte Seitenkette einer natürlich vorkommenden α-Aminosäure steht, wie z.B. geschütztes Ser, Thr, Asp, Asn, Glu, Gln, Arg, Lys, Hyl, Cys, Orn, Cit, Tyr, Trp, His oder Hyp, sind als Schutzgruppen die in der Peptidchemie üblichen Gruppen bevorzugt (vgl. Houben-Weyl, Bd. XV/1 und XV/2)). Im Falle, daß $R^1$ die geschützte Lysin-Seitenkette bedeutet, werden die bekannten Aminoschutzgruppen, insbesondere aber ($C_1$—$C_6$)-Alkanoyl bevorzugt. Im Falle, daß $R^1$ die geschützte Tyrosin-Seitenkette bedeutet, wird die -Ether-Schutzgruppe am Sauerstoff, insbesondere ($C_1$—$C_6$)-Alkyl, bevorzugt; besonders bevorzugte Schutzgruppen sind Methyl und Ethyl.

Verbindungen der Formel I besitzen chirale C-Atome. Sowohl die R- als auch die S-Konfigurationen an allen Asymmetriezentren sind Gegenstand der Erfindung. Die Verbindungen der Formel I können daher als optische Isomere, als Diasteromere, als Racemate oder als Gemische derselben vorliegen. Bevorzugt sind jedoch die Verbindungen der Formel I, in denen das C-Atom 3 im bicyclischen Ringsystem, sowie die mit einem Stern (*) markierten C-Atome der Seitenkette S-Konfiguration aufweisen, mit der Ausnahme, daß bei (NH—CHR$^1$—CO) = Cys die R-Konfiguration dieses Zentrums bevorzugt ist.

Die Erfindung betrifft ferner ein Verfahren zur Herstellung der Verbindungen der Formel I, das dadurch gekennzeichnet ist, daß man eine Verbindung der Formel II, in der n, $R^1$, $R^2$, X, Y und Z die vorstehend genannten Bedeutungen mit Ausnahme der von $R^2$ = Wasserstoff hat, mit einer Verbindung der Formeln IIIa oder IIIb oder dem Spiegelbild oder dem Racemat, in denen W eine Carboxy veresternde Gruppe, wie ($C_1$—$C_6$)-Alkyl oder ($C_7$—$C_8$)-Aralkyl, vorzugsweise tert.-Butyl oder Benzyl bedeutet, nach bekannten Amidbildungsmethoden der Peptidchemie umsetzt und anschließend durch Hydrierung oder Säure- oder/ und Basenbehandlung die Verbindungen des Types I mit R = Wasserstoff freisetzt.

Diastereomere der Formel I können beispielsweise durch Kristallisation oder Chromatographie voneinander getrennt werden.

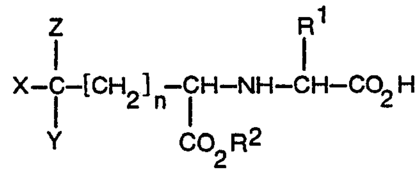

(II)

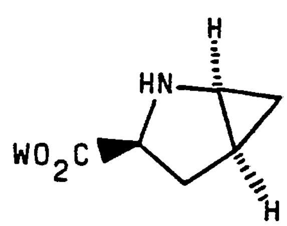

(IIIa) und Spiegelbild

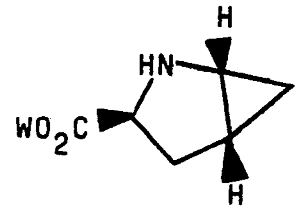

(IIIb) und Spiegelbild

Weitere Syntheseverfahren zur Herstellung der Verbindungen der Formel I, in der Y und Z zusammen Sauerstoff bedeuten, bestehen darin, daß man eine Verbindung der Formel IV

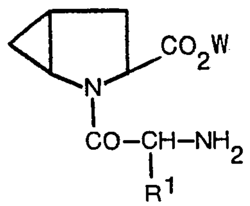

(IV)

in welcher $R^1$ die Bedeutung wie in Formel I und W die Bedeutung wie in den Formeln III a und b besitzen, mit einer Verbindung der Formel V

$$R^2O_2C—CH=CH—CO—X \qquad (V)$$

worin $R^2$ und X die Bedeutungen wie in Formel I besitzen, in bekannter Weise in einer Michael-Reaktion (Organikum, 6. Auflage, S. 492, 1967) umsetzt und gegebenenfalls den Rest W und/oder den Rest $R^2$, wie oben beschrieben, abspaltet oder daß man eine Verbindung der obengenannten Formel IV mit einer Verbindung der allgemeinen Formel VI, worin $R^2$ die Bedeutung wie in Formel I hat, und mit einer Verbindung der allgemeinen Formel VII

$$OHC—CO_2R^2 \qquad (VI)$$

$$X—CO—CH_3 \qquad (VII)$$

worin X die Bedeutung wie in Formel I hat, in bekannter Weise in einer Mannich-Reaktion (Bull. Soc. Chim. France 1973, S. 625) umsetzt und anschließend gegebenenfalls den Rest W und/oder den Rest $R^2$ wie oben beschrieben unter Bildung der freien Carboxygruppen abspaltet.

Ferner können Verbindungen der Formel I mit Y und Z jeweils = Wasserstoff auch in der Weise hergestellt werden, daß man eine Verbindung der oben genannten Formel IV gemäß der in J. Amer. Chem. Soc. *93*, 2897 (1971) beschriebenen Verfahrensweise mit einer Verbindung der Formel VIII

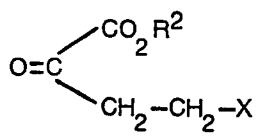

(VIII)

worin $R^2$ und X die Bedeutungen wie in Formel I besitzen, umsetzt, die erhaltenen Schiff-Basen reduziert und anschließend gegebenenfalls den Rest W und/oder den Rest $R^2$, wie oben beschrieben, unter Bildung der freien Carboxygruppen abspaltet. Die Reduktion der Schiff-Basen kann elektrolytisch oder mit Reduktionsmittel wie beispielsweise Natriumborhydrid oder Natriumcyanborhydrid erfolgen.

Verbindungen der Formel I mit Y = Hydroxy und Z = Wasserstoff können beispielsweise auch durch Reduktion einer gemäß obigen Verfahrensweisen erhaltenen Verbindung I mit Y und Z = zusammen Sauerstoff erhalten werden. Diese Reduktion kann mit einem Reduktionsmittel wie Natriumborhydrid und anderen komplexen Boranaten oder beispielsweise Boran-Amin-Komplexen, erfolgen.

Verbindungen der Formel I, in welcher R für Wasserstoff steht, können gegebenenfalls nach an sich bekannten Methoden in ihre Ester der Formel I, worin R ($C_1$ bis $C_6$)-Alkyl oder ($C_7$ bis $C_9$)-Aralkyl bedeutet, überführt werden.

Die Umsetzung einer Verbindung der Formel II mit einer Verbindung der Formel III zur Herstellung einer Verbindung der Formel I erfolgt gemäß einer in der Peptidchemie bekannten Kondensationsreaktion, wobei als Kondensationsmittel beispielsweise Dicyclohexylcarbodiimid und 1-Hydroxy-benzotriazol zugesetzt werden. Bei einer nachfolgenden sauren Abspaltung des Restes W werden als Säuren bevorzugt Trifluoressigsäure oder Chlorwasserstoff eingesetzt.

Verbindungen der Formel II sind bereits vorgeschlagen worden. Solche mit X = Phenyl, Y = H, Z = H, $R^1 = CH_3$ und $R^2 = CH_3$ oder $C_2H_5$ sind bekannt (z.B. aus der EP-Schr. 0037 231) und auf verschiedenen Wegen zugänglich. Die Benzylester ($R^2$ = Benzyl) können analog hergestellt werden.

Die Mannich-Reaktion von Acetophenonen der Formeln IXa, in der X für gegebenenfalls wie vorstehend substituiertes Aryl steht, mit Glyoxylsäureestern und α-Aminosäureestern führt zu Verbindungen der Formel II, in denen n = 1 und Y und Z zusammen Sauerstoff bedeuten (Formel IX). In der Formel IX bedeutet W' einen hydrogenolytisch, basisch oder sauer abspaltbaren Rest, bevorzugt Benzyl oder tert.Butyl, X steht für die gegebenenfalls wie vorstehend definierten Bedeutungen.

Im Falle des Benzylesters (W' = Benzyl) darf jedoch $R^2$ nicht Benzyl sein. Bei der Hydrogenolyse dieser Verbindungen mit Pd entstehen Verbindungen der Formel II, in denen Y und Z Wasserstoff sind.

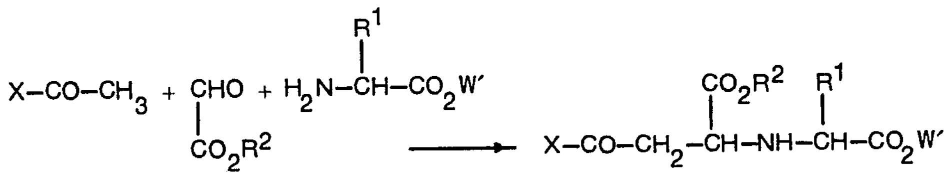

(IXa) (IX)

Verbindungen der Formel II in der Y und Z zusammen Sauerstoff bedeuten, können ebenfalls durch Michael-Addition entsprechender Acyl-Acrylsäureester mit α-Aminosäureestern in hohen Ausbeuten gewonnen werden. Esterspaltung führt zu denselben Produkten wie die Mannich-Reaktion.

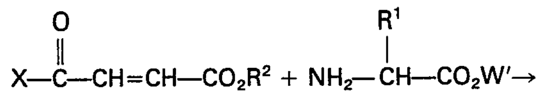 (IX)

Die Diastereomeren mit der bevorzugten S,S-Konfiguration entstehen bei Einsatz von Estern der L-Aminosäuren in überwiegender Menge und können durch Kristallisation oder chromatographische Trennung der Ester an Kieselgel gewonnen werden.

Die als Ausgangsstoffe zur Herstellung der Verbindungen der Formel I verwendeten Verbindungen der obengenannten Formel IV werden aus den Verbindungen der obengenannten Formeln III a oder b oder der Spiegelbilder durch Umsetzen mit einer N-geschützen 2-Aminocarbonsäure der Formel X

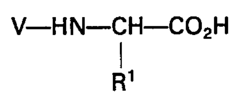 (X)

worin V eine Schutzgruppe und $R^1$ die obengenannte Bedeutung besitzt, nach bekannter Verfahrensweise erhalten. Als Schutzgruppe V, die nach beendeter Reaktion wieder abgespalten wird, kommt beispielsweise tert.-Butoxycarbonyl in Frage.

Die Erfindung betrifft Verbindungen der Formeln IIIa und IIIb und deren Spiegelbilder, in welchen W Wasserstoff oder eine Carboxy veresternde Gruppe, vorzugsweise ($C_1$ bis $C_6$)-Alkyl oder ($C_7$ oder $C_8$)-Aralkyl bedeutet, sowie ein Verfahren zu deren Herstellung, das dadurch gekennzeichnet ist, daß Verbindungen der Formel XI

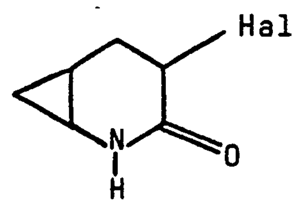 (XI)

in welcher Hal für Halogen, vorzugsweise Chlor oder Brom steht, in Gegenwart einer Base umlagert und die erhaltenen Verbindungen der Formeln IIIa oder IIIb (W = Wasserstoff) und/oder deren Spiegelbilder gegebenenfalls in an sich bekannter Weise in die oben genannten Ester überführt.

Verbindungen der allgemeinen Formel XI können hergestellt werden, indem man eine Verbindung der Formel XII (Limasset et al., Bull. Soc. Chim. France *1969*, 3981)

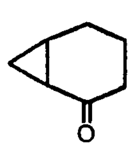 (XII)

in ihr Oxim überführt, dieses in einer Beckmann-Umlagerung z.B. analog Helv. Chim. Acta *46*, 1190, (1963) zu einer Verbindung der Formel XIII

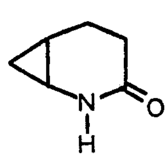 (XIII)

umsetzt, diese zu einer Verbindung der Formel XIV,

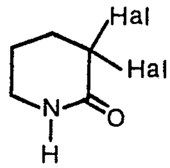

(XIV)

in welcher Hal ein Halogenatom, vorzugsweise Chlor oder Brom bedeutet, halogeniert und diese katalytisch zu Verbindungen der Formel XI reduziert.

Zur Überführung des Ketons XII in das entsprechende Oxim arbeitet man gewöhnlich in wäßrigalkoholischem Medium mit überschüßigem Hydroxylamin-hydrochlorid, wobei die freie Säure mit Soda oder Natriumacetat abgestumpft wird. Statt des Hydroxylamins, läßt sich auch Hydroxylamin-N,N-disulfonsaures Natrium (Org. Synth. *3* [1923] 61) oder das Natriumsalz der Hydroxylamin-N-monosulfonsäure (J. Amer. Chem. Soc. *46* [1924] 1290) verwenden.

Als besonders vorteilhaft hat sich die Umsetzung des Ketons XII mit Hydroxylamin-O-sulfonsäure in einer konzentrierten organischen Säure, vorzugsweise Ameisensäure erwiesen, wobei das Oxim in situ erzeugt und ohne Isolierung in die Verbindung XIII umgelagert wird, die zusammen mit ihrem Isomeren der Formel XIII a entsteht.

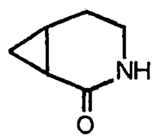

(XIIa)

Als Halogenierungsmittel kommen beispielsweise anorganische Säurehalogenide wie $PCl_5$, $SO_2Cl_2$, $POCl_3$, $SOCl_2$, $PBr_3$ oder Halogene wie Brom oder Chlor in Betracht. Von Vorteil ist die Verwendung von $PCl_5$ oder $POCl_3$ in Kombination mit $SO_2Cl_2$ in einem organischen Lösemittel. Als Zwischenstufe bildet sich zunächst ein Imidhalogenid, das mit den genannten Halogenierungsmitteln und anschließender Hydrolyse unter basischen Bedingungen, vorzugsweise mit wäßrigem Alkalicarbonat weiter zu einer Verbindung der Formel XIV reagiert.

Die Verbindungen der Formel XIV werden nachfolgend in einem polaren protischen Lösemittel wie beispielsweise einem Alkohol, vorzugsweise Ethanol, oder einer Carbonsäure wie beispielsweise Essigsäure unter Zusatz eines Säureacceptors wie beispielsweise Natriumacetat oder Triethylamin zu einer Verbindung der Formel XI, worin Hal die oben genannte Bedeutung besitzt, katalytisch reduziert. Als Katalysator kommen beispielsweise Raney-Nickel oder Palladium bzw. Platin auf Tierkohle in Betracht.

Verbindungen der Formel XI können auch direkt oder im Gemisch mit Verbindungen der Formel XIV durch Halogenierung der Verbindungen der Formel XIII beim Einsatz geringerer Mengen der obengenannten Halogenierungsmittel hergestellt werden.

Verbindungen der Formel XI werden gemäß der bekannten Favorskii-Reaktion in Gegenwart einer Base zu den Verbindungen der Formeln III a oder b mit W = Wasserstoff umgesetzt und diese gegebenenfalls verestert. Die obengenannte Favorskii-Reaktion wird in einem alkoholischen Lösungsmittel wie Methanol, Ethanol oder tert.-Butanol oder in Wasser oder in Gemischen derselben bei Temperaturen im Bereich von 20° bis 140°C, vorzugsweise zwischen 60° und 100°C durchgeführt. Als Basen werden zweckmäßig Alkali- oder Erdalkalihydroxide wie Natrium-, Kalium- oder Bariumhydroxid oder Alkalialkoholate wie beispielsweise Natriummethylat oder Kalium-tert.-butanolat eingesetzt.

Die gemäß oben beschriebener Verfahrensweise erhaltenen Verbindungen der Formel IIIa und IIIb oder deren Spiegelbilder fallen als Stereoisomerengemische an und können beispielsweise durch Umkristallisieren oder durch Chromatographie voneinander getrennt werden. Gegebenenfalls müssen die Gemische entsprechend derivatisiert werden, um sie dann durch Umkristallisation oder durch Chromatographie voneinander zu trennen.

Racemische Gemische von Verbindungen der Formeln III a bzw. IIIb können als solche in die weiteren oben beschriebenen Synthesen eingesetzt werden. Sie können jedoch auch vor weiteren Umsetzungen, falls es gewünscht wird, nach den bekannten Methoden der Racematspaltung (vgl. z.B. Quart. Rev. *25* (1971) 323 ff.) in die Enantiomeren getrennt werden.

Fallen die Verbindungen der Formel I als Racemate an, können auch diese nach den üblichen Methoden wie beispielsweise über Salzbildung mit optisch aktiven Basen oder Säuren in ihre Entantiomeren gespalten oder durch Chromatographie getrennt werden.

Die erfindungsgemäßen Verbindungen der Formel I, liegen falls R = Wasserstoff ist, als innere Salze vor. Als amphotere Verbindungen können sie Salze mit Säuren oder Basen bilden. Diese Salze werden in üblicher Weise durch Umsetzen mit einem Äquivalent Säure bzw. Base hergestellt.

Die Verbindungen der Formel I und deren Salze besitzen lang andauernde, intensive blutdrucksenkende Wirkung. Sie sind starke Hemmer des Angiotensin-Converting-Enzyms (ACE-Hemmer) und können zur Bekämpfung des Bluthochdrucks verschiedener Genese eingesetzt werden. Auch ihre Kombination mit anderen blutdrucksenkenden, gefäßerweiternden oder diuretisch wirksamen Verbindungen sind möglich. Typische Vertreter dieser Wirkklassen sind z.B. in Erhardt-Ruschig,

Arzneimittel, 2. Auflage, Weinheim, 1972, beschrieben. Die Anwendung kann intravenös, subeutan oder peroral erfolgen.

Die Dosierung bei oraler Gabe liegt bei 1—100 mg, vorzugsweise 1—50, insbesondere 1—30 mg je Einzeldosis für einen normalgewichtigen Erwachsenen. Dies entspricht ca. 13—1300 µg/kg/Tag, vorzugsweise 13—650 µg/kg/Tag, insbesondere 13—400 µg/kg/Tag. Die Dosis kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfingungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige alkoholische oder ölige Suspensionen oder wäßrige alkoholische oder ölige Lösungen. Als inerte Träger können z.B. Gummi arabicum, Magnesiumcarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

Die nachfolgenden Beispiele sollen die erfindungsgemäßen Verfahrensweisen erläutern, ohne die Erfindung auf die hier stellvertretend genannten Substanzen zu besehränken.

Die $^1H$—NMR-Daten sind δ-Werte.

Beispiel I

N-(1-S-Carbethoxy-3-phenyl-propyl)-S-alanyl-cis-2-azabicyclo[3,1,0]hexan-3-carbonsäurehydrochlorid

(1) Cis-2-azabicyclo[4,1,0]-3-oxo-heptan

1 g Cis-bicyclo[(3,1,0]-2-oxo-hexan werden in 10 ml 97%iger Ameisensäure gelöst. Unter Eiskühlung werden 1,8 g Hydroxylamin-O-sulfonsäure in 5 ml 97%iger Ameisensäure zugegeben und anschließend 45 Minuten auf Rückflußtemperatur gebracht. Nach dem Abkühlen wird auf Eis gegossen, mit festem $NaHCO_3$ neutralisiert und mit Essigester extrahiert. Nach dem Trocknen und Eindampfen bleibt ein Rückstand von 1 g Öl.

(2) Cis-2-azabicyclo[4,1,0]-3-oxo-4-dichlor-heptan

11 g des nach Beispiel I (1) erhaltenen Rohprodukts werden in 250 ml Dichloräthan gelöst. Unter Eiskühlung werden 20.8 g Phosphorpentachlorid eingetragen. Es wird 30 Minuten bei Raumtemperatur gerührt. Anschließend werden utner Eiskühlung 17 ml Sulfurylchlorid zugetropft, 1 Stunde bei Raumtemperatur und 5 Stunden bei 60°C (Badtemperatur) unter Stickstoff gerührt. Nach dem Abkühlen wird mit 200 g Eis versetzt, mit festem Natriumcarbonat neutralisiert, die Dichloräthanphase abgetrennt, die wäßrige Phase mit Methylenchlorid nachextrahiert. Die oranischen Phasen werden nach dem Trocknen eingedampft und der Rückstand (20 g) über Kieselgel mit Methylenchlorid/Essigester 19:1 Elutionsmittel chromatographiert.

Ausbeute: 4,2 g    Fp: 174—175°C

(3) Cis-2-azabicyclo[4,1,0]-3-oxo-4-chlor-heptan

3,0 I(2) werden in Ethanol gelöst, 2,8 ml Triäthylamin und 2,5 g Raney-Nickel zugegeben und 20 min hydriert. Es wird vom Katalysator abgesaugt, eingedampft und der Rückstand über Kieselgel mit Methylenchlorid und Essigester 19:1 als Elutionsmittel chromatographiert.

Ausbeute: 1,8 g

Analyse: $C_8H_8NOCl$:

| | | | | |
|---|---|---|---|---|
| bwe. | 49,5 | 5,5 | 9,6 | 24,3 |
| gef. | 49,8 | 5,2 | 9,3 | 23,9 |

(4a) Cis-2-azabicyclo[3,1,0]hexan-3-carbonsäure

1.6 g I(3) werden in 55 ml Wasser suspendiert und 4,0 g $(Ba(OH)_2.8\ H_2O$ zugegeben. Nach 1 Stunde efluxieren wird mit 2 n Schwefelsäure auf pH 2,5 gestellt, vom Niederschlag abgesaugt, die wäßrige Lösung auf pH 6 gebracht, zur Trockene eingedampft, mit Ethanol und Methylenchlorid versetzt, vom Niederschlag abgesaugt, die Lösung eingeengt und der Rückstand mit Essigester versetzt. Ausbeute 1,5 g farbloses festes Produkt.

$^1H$—NMR/60 MHz, $D_2O$:
0,4—1,2 (m, 2 H);
1,5—2,9 (m, 3 H);
3,1—4,4 (m, 2 H)

(4b) Cis-2-azabicyclo[3,1,0]hexan-endo/exo-3-carbonsäure

Das in Bespiel I(4a) erhaltene Produkt besteht aus einem cis-endo/cis-exo-Gemisch. Dieses kann nach Derivatisierung zum Benzylester und anschließender N-Acylierung mit Chlorameisensäurebenzylester mittels Kieselgelchromatographie in das Cis-endo- und das cis-exo-Derivat getrennt werden. Durch anschließende Hydrierung mit Pd/C (10%) als Katalysator erhält man die rac. cis-endo bzw. cis-exo-Aminosäure.

$^1H$—NMR der Cis-exo-2-azabicyclo[3,1,0]hexan-3-carbonsäure
(270 MHZ, $D_2O$):
0,8—0,93 (m, 2 H);
1,8—1,92 (m, 1 H);
2,1—2,24 (m, 1 H);
2,45—2,55 (qu, 1 H);
3,29—3,37 (m, 1 H);
3,78—3,88 (d dn, 1 H)

$^1H$—NMR der Cis-exo-2-azabicyclo[3,1,0]hexan-3-carbonsäure
(270 MHZ, $D_2O$):
0,56—0,66 (m, 1 H);
1,87—0,99 (aufgesp. qu. 1 H)
1,79—1,9 (m, 1 H);
2,32—2,41 (m, 1 H);
2,48—2,62 (m, 1 H);
3,30—3,39 (m, 1 H);
4,26—4,35 (aufgesp. dn, 1 H)

Die diastereomerenreinen Produkte oder das Gemisch kann in die weiteren Reaktionen eingesetzt werden.

(5a) Cis-2-azabicyclo[3,1,0]hexan-endo/exo-3-carbonsäurebenzylester

Bei −15°C werden zu 30 ml Benzylalkohol 1,22 ml Thionylchlorid zugetropft. Anschließend werden bei −10°C 1,5 g der in Beispiel I(4a) hergestellten cis-endo/exo-Aminosäuregemisches eingetragen. Nach einer Reaktionszeit von 24 Stunden bei Raumtemperatur wird mit Ether verdünnt und unter Eiskühlung mit Wasser ausgerührt. Die wäßrige Lösung wird mit $NaHCO_3$ neutralisiert und mit Ether/Methylenchlorid extrahiert, nach dem Trocknen wird eingedampft.

Ruckstand 2,2 g Öl.

$R_f$: 0,65 (Kieselgel, Methylenchlorid, Methanol, Eisessig, Wasser 20:10:2:2, Ninhydriunanfärbung)

(5b) Cis-2-azabicyclo[3,1,0]hexan-endo-3-carbonsäurebenzylester

Man erhält diese Verbindung durch Umsetzung der in Beispiel I (4b) hergestellten cis-exo-Aminosäure mit Benzylalkohol und Thionylchlorid nach dem in Beispiel I(5a) angegebenen Verfahren.

$R_f$: 0,62 (Kieselgel, Methylenchlorid, Methanol, Eisessig, Wasser 20:10:2:2, Ninhydrinanfärbung)

(5c) Cis-2-azabicyclo[3,1,0]hexan-endo-3-carbonsäurebenzylester

Man erhält diese Verbindung durch Umsetzung der in Beispiel I (4b) hergestellten cis-endo-Aminosäure mit Benzylalkohol und Thionylchlorid nach dem in Beispiel I(5a) angegebenen Verfahren.

$R_f$: 0,69 (Kieselgel, Methylenchlorid, Methanol, Eisessig, Wasser 20:10:2:2, Ninhydrinanfärbung)

(6) N-(1-S-Carbethoxy-3-phenyl-propyl)-S-alanyl-cis-2-azabicyclo[3,1,0]hexan-exo/endo-3-carbonsäurebenzylester

2,0 g des nach Beispiel I(5a) hergestellten Benzylesters werden mit 1,4 g Hydroxybenztriazol (HOBt), 1,76 g Dicyclohexylcarbodiimid und 2,2 g N-(1-S-Carbethoxy-3-phenylpropyl)-S-alanin in 28 ml Dimethylformamid zur Reaktion gebracht. Nach 10 Stunden Rühren bei Raumtemperatur saugt man vom ausgefallenen Dicyclohexylharnstoff ab, engt ein, nimmt in Methylenchlorid auf und schüttelt 2 × mit gesättigter $NaHCO_3$-Lösung aus. Die organische Phase wird nach dem Trocknen eingeengt und 5,4 g des cis-exo/cis-endo-Diastereomerengemische erhalten.

(7) N-(1-S-Carbethoxy-3-phenyl-propyl)-S-alanyl-cis-2-azabicyclo[3,1,0]hexan-endo-3-S-carbonsäurebenzylester (Isomer 1)

Das in Beispiel I(6) erhaltene Rohprodukt wird mit Cyclohexan/Essigester im Verhältnis 1,5:1 über

Kieselgel chromatographiert. Das zuerst eluierte Isomer stellt die S,S,S cis-endo-Verbindung dar.

$R_f$: 0,24 (Kieselgel, Cyclohexan/Essigester 1:1)

$^1$H—NMR ($CDCl_3$):

0,6—3,0 (m 13 H);
3,2—3,9 (m 4 H);
3,95—4,4 (q, 4 H);
4,8—5,0 (Doppeltes Dublett, 1 H)
5,15 (s, 2 H);
7,25 (s, 5 H);
7,35 (s, 5 H)

(8) N-(1-S-Carbethoxy-3-phenyl-propyl)-S-alanyl-cis-2-azabicyclo[3,1,0]hexan-endo-3-R-carbonsäurebenzylester (Isomer 2)

Das in Beispiel I(6) erhaltene Rohprodukt wird mit Cyclohexan/Essigester im Verhältnis 1,5:1 über eine Säule aus Kieselgel chromatographiert. Das an vierter Stelle eluierte Isomere stellt die cis-endo-S,S,R-Verbindung dar.

$R_f$: 0,09 (Kieselgel, Cyclohexan/Essigester 1:1)

(9) N-(1-S-Carbethoxy-3-phenyl-propyl)-S-alanyl-cis-2-azabicyclo[3,1,0]hexan-exo-3-S-carbonsäurebenzylester (Isomer 3)

Das in Beispiel I(6) erhaltene Rohprodukt wird mit Cyclohexan/Essigester im Verhältnis 1,5:1 über eine Kieselgelsäule chromatographiert. Das an zweiter Stelle eluierte Isomere stellt die cis-exo-S,S,S-Verbindung dar.

$R_f$: 0,20 (Kieselgel, Cyclohexan/Essigester 1:1)

(10) N-(1-S-Carbethoxy-3-phenyl-propyl)-S-alanyl-cis-2-azabicyclo[3,1,0]hexan-exo-3-R-carbonsäurebenzylester (Isomer 4)

Das in Beispiel I(6) erhaltene Rohprodukt wird mit Cyclohexan/Essigester im Verhältnis 1,5:1 über eine Kieselgelsäule chromatographiert. Das an dritter Stelle eluierte Isomere stellt die cis-exo-S,S,R-Verbindung dar.

$R_f$: 0,14 (Kieselgel, Cyclohexan/Essigester 1:1)

(11) N-(1-S-Carbethoxy-3-phenyl-propyl)-S-alanyl-cis-2-azabicyclo[3,1,0]hexan-3-carbonsäurehydrochlorid

1,0 g des in Beispiel I(6) hergestellten Benzylesters wird als Diastereomerengemisch in 50 ml Ethanol gelöst. Dazu werden 100 mg Palladium auf Kohle (10%) gegeben und bei Raumtemperatur und unter Normaldruck hydriert. Nach dem Absaugen des Katalysators wird die ethanolische Lösung eingedampft, das zurückbleibende Öl wird mit ethanolischer Salzsäure versetzt, das Lösungsmittel abgedampft. Der Rückstand wird mit Essigester verrührt, wobei ein farbloser Feststoff als Diastereomerengemisch der exo/endo-Carbonsäure (Hydrochlorid) zurückbleibt.

Ausbeute: 0,7 g

## Beispiel II

### N-(1-S-Carbethoxy-3-phenyl-propyl)-S-alanyl-cis-2-azabicyclo[3,1,0]hexan-endo-3-S-carbonsäurehydrochlorid (Isomer I)

0,2 g des in Beispiel I(7) hergestellten Benzylesters werden analog Beispiel I(11) katalytisch entbenzyliert und in das Hydrochlorid überführt.

Ausbeute: 0,125 g

$^1$H—NMR (DMSO-$d_6$):

0,6—5,0 (m, 21 H);
7,3 (s, 5 H);
9,7 (sehr breites s)

## Beispiel III

### N-(1-S-Carbethoxy-3-phenyl-propyl)-S-alanyl-cis-2-azabicyclo[3,1,0]hexan-endo-3-S-carbonsäurehydrochlorid (Isomer I)

0,2 g des in Beispiel I(7) hergestellten Benzylesters werden analog Beispiel I(11) katalytisch entbenzyliert und in das Hydrochlorid überführt.

Ausbeute: 0,125 g

$^1$H—NMR (DMSO-$d_6$):

0,6—5,0 (m, 21 H);
7,3 (s, 5 H);
9,7 (sehr breites s)

Beispiel III

N-(1-S-Carbethoxy-3-phenyl-propyl)-S-alanyl-cis-2-azabicyclo[3,1,0]hexan-endo-3-R-carbonsäurehydrochlorid (Isomer 2)

0,22 g des in Beispiel I(8) hergestellten Benzylesters werden analog Beispiel I(11) katalytisch entbenzyliert und in das Hydrochlorid überführt.

Ausbeute: 0,13 g

$^1H$—NMR (DMSO-$^d{}_6$):

0,5—5,1 (m, 21 H);

7,2 (s, 5 H);

10,1 (sehr breites s)

Beispiel IV

N-(1-S-Carbethoxy-3-phenyl-propyl)-S-alanyl-cis-2-azabicyclo[3,1,0]hexan-exo-3-S-carbonsäurehydrochlorid (Isomer 3)

0,27 g des in Beispiel I(9) hergestellten Benzylesters werden analog Beispiel I(11) katalytisch entbenzyliert und in das Hydrochlorid überführt.

Ausbeute: 0,23 g

$^1H$—NMR (DMSO-$^d{}_6$):

0,5—4,7 (m, 21 H);

7,3 (s, 5 H);

10,0 (sehr breites s)

Beispiel V

N-(1-S-Carbethoxy-3-phenyl-propyl)-S-alanyl-cis-2-azabicyclo[3,1,0]hexan-exo-3-R-carbonsäurehydrochlorid (Isomer 4)

0,3 g des in Beispiel I(10) hergestellten Benzylesters werden analog Beispiel I(11) katalytisch entbenzyliert und in das Hydrochlorid überführt.

Ausbeute: 0,26 g

$^1H$—NMR (DMSO-$^d{}_6$):

0,4—4,8 (m, 21 H);

7,3 (s, 5 H);

9,8 (sehr breites s)

Beispiel VI

N-(1-S-Carboxy-3-phenyl-propyl)-S-alanyl-cis-2-azabicyclo[3,1,0]hexan-endo-3-S-carbonsäure (Isomer 1)

0,2 g des Ethylesteraus aus Beispiel II werden in 5 ml Wasser gelöst. Die Lösung wird mit 4 n wäßrige Kaliumhydroxydlösung basisch gestellt. Es wird über Nacht bei 0°C gelassen. Nach dem Ende der Reaktion wird mit konz. Salzsäure auf pH 5 gebracht. Die Lösung wird auf 20 ml stark sauren Ionenaustauscher (IR 120, $H^+$-Form) aufgetragen, mit Wasser entwickelt und mit Wasser, das 2% Hydrin enthält, eluiert. Die obige Verbindung enthaltende Fraktionen werden eingedampft. Der Rückstand wird 2 × mit Toluol behandelt und vom Toluol unter vermindertem Druck befriet. Der Rückstand wird mit Ether versetzt.

Ausbeute: 0,13 g

$^1H$—NMR ($D_2O$):

0,6—5,1 (m, 15 H);

7,3 (s, 5 H)

Beispiel VII

N-(1-S-Carbethoxy-3-phenyl-propyl)-S-alanyl-cis-2-azabicyclo[3,1,0]hexan-exo-3-S-carbonsäure (Isomer 3)

0,2 g des Ethylesters aus Beispiel IV werden analog Beispiel VI verseift und aufgearbeitet.

Ausbeute: 0,12 g

$^1H$—NMR ($D_2O$):

0,4—4,8 (m, 16 H);

7,2 (s, 5 H)

**Patentansprüche für die Vertragsstaaten: BE CH DE FR GB IT LI LU NL SE**

1. Verbindung der Formel I,

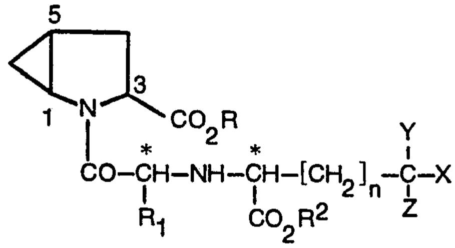

(I)

in der die Wasserstoffatome an den Brückenkopf-C-Atomen zueinander cis- konfiguriert sind und die COOR-Gruppe am C-Atom 3 exo- oder endoständig zum bicycloischen Ringsystem orientiert ist, in der

n = 0 oder 1,

R = Wasserstoff, ($C_1$—$C_6$)-Alkyl, ($C_2$—$C_6$)-Alkenyl oder ($C_6$—$C_{12}$)-Aryl-($C_1$—$C_4$)-Alkyl,

$R^1$ = Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_5$ bis $C_9$)-Cycloalkyl, ($C_5$ bis $C_9$)- Cycloalkenyl, ($C_5$ bis $C_7$)-Cycloalkyl-($C_1$ bis $C_4$)-alkyl, ($C_6$ bis $C_{12}$)-Aryl oder teilhydriertes ($C_6$ bis $C_{12}$)-Aryl, das jeweils durch ($C_1$ bis $C_4$)-Alkyl, ($C_1$ oder $C_2$)-Alkoxy oder Halogen substituiert sein kann, ($C_6$ bis $C_{12}$)-Aryl-($C_1$ bis $C_4$)-alkyl oder ($C_7$ bis $C_{13}$)-Aroyl-($C_1$—$C_2$)alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden α-Aminosäure $R^1$—CH($NH_2$—COOH,

$R^2$ = Wasserstoff, ($C_1$—$C_6$)-Alkyl, ($C_2$—$C_6$)-Alkenyl oder ($C_6$—$C_{12}$)-Aryl-($C_1$—$C_4$)-alkyl,

Y = Wasserstoff oder Hydroxy,

Z = Wasserstoff oder

Y und Z = zusammen Sauerstoff,

X = ($C_1$—$C_6$)-Alkyl, ($C_2$—$C_6$)-Alkenyl, ($C_5$-$C_9$)-Cycloalkyl, ($C_6$—$C_{12}$)-Aryl, das durch ($C_1$—$C_4$)-Alkyl, ($C_1$—$C_4$)-Alkoxy, Hydroxy, Halogen, Nitro, Amino, ($C_1$—$C_4$)-Alkylamino, Di-($C_1$—$C_4$)-alkyl-amino und/oder Methylendioxy mono-, di- oder trisubstituiert sein kann, oder Indol-3-yl bedeuten, sowie deren physiologisch unbedenkliche Salze.

2. Verbindung der Formel I gemäß Anspruch 1, dadurch gekennzeichnet, daß das C-Atom in Position 3 des bicyclischen Ringsystems sowie die mit einem Stern markierten C-Atome der Seitenkette jeweils S-Konfiguration aufweisen.

3. Verbindung der Formel I gemäß einem der Ansprüche 1 oder 2, in welcher

n = 1 ist,

$R^1$ = Wasserstoff, Allyl, Vinyl oder eine gegebenfalls geschützte Seitenkette einer natürlich vorkommenden α-Aminosäure bedeutet und

R, $R^2$, Y, Z und X die in Anspruch 1 definierten Bedeutungen haben.

4. Verbindung der Formel I gemäß einem der Ansprüche 1 bis 3, in welcher

n = 1,

R = Wasserstoff

$R^1$ = Methyl, die gegebenenfalls acylierte Seitenkette von Lysin oder die O—($C_1$—$C_6$)-alkylierte Seitenkette ders Tyrosin,

$R^2$ = Wasserstoff, Methyl, Ethyl, Benzyl oder tert. Butyl

X = Phenyl oder mit Fluor und/oder Chlor mono- oder disubstituiertes Phenyl,

Y = Wasserstoff oder Hydroxy

Z = Wasserstoff oder

Y und Z zusammen Sauerstoff bedeuten.

5. N-(1-S-Carbethoxy-3-phenyl-propyl)-S-alanyl-cis-2-azabicyclo[3,1,0]hexan-endo-3-S-carbonsäure,

6. N-(1-S-Carboxy-3-phenyl-propyl)-S-alanyl-cis-2-azabicyclo[3,1,0]hexan-endo-3-S-carbonsäure,

7. N-(1-S-Carbethoxy-3-phenyl-propyl)-S-lysyl-cis-2-azabicyclo[3,1,0]hexan-endo-3-S-carbonsäure,

8. N-(1-S-Carboxy-3-phenyl-propyl)-S-lysyl-cis-2-azabicyclo[3,1,0]hexan-endo-3-S-carbonsäure,

9. N-(1-S-Carbethoxy-3-phenyl-propyl)-O-ethyl-S-tyrosyl-cis-2-azabicyclo[3,1,0]hexan-endo-3-S-carbonsäure,

10. N-(1-S-Carbethoxy-3-phenyl-propyl)-O-methyl-S-tyrosyl-cis-2-azabicyclo[3,1,0]hexan-endo-3-S-carbonsäure,

11. N-(1-S-Carbethoxy-3-phenyl-propyl)-S-alanyl-cis-2-azabicyclo[3,1,0]hexan-exo-3-S-carbonsäure,

12. N-(1-S-Carboxy-3-phenyl-propyl)-S-alanyl-cis-2-azabicyclo[3,1,0]hexan-exo-3-S-carbonsäure,

13. N-(1-S-Carbethoxy-3-phenyl-propyl)-S-lysyl-cis-2-azabicyclo[3,1,0]hexan-exo-3-S-carbonsäure,

14. N-(1-S-Carboxy-3-phenyl-propyl)-S-lysyl-cis-2-azabicyclo[3,1,0]hexan-endo-3-S-carbonsäure,

15. N-(1-S-Carbethoxy-3-phenyl-propyl)-O-ethyl-S-tyrosyl-cis-2-azabicyclo[3,1,0]hexan-exo-3-S-carbonsäure,

16. N-(1-S-Carbethoxy-3-phenyl-propyl)-O-methyl-S-tyrosyl-cis-2-azabicyclo[3,1,0]hexan-exo-3-S-carbonsäure,

17. Verfahren zur Herstellung von Verbindungen gemäß den Ansprüchen 1 bis 16, dadurch gekennzeichnet,

a) daß man eine Verbindung der Formel II

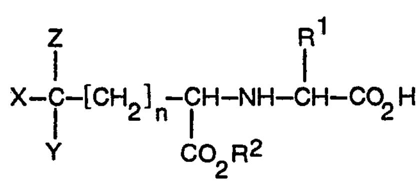

(II)

in der n, X, Y, Z, $R^1$ und $R^2$ die im Anspruch 1 definierten Bedeutungen mit Ausnahme von $R^2$ = Wasserstoff haben, mit einer Verbindung der Formeln IIIa oder IIIb oder dem Stereoisomerengemisch

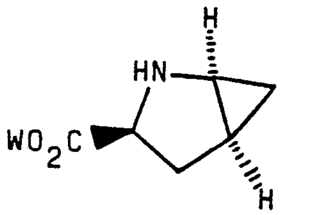

(IIIa) mit Spiegelbild

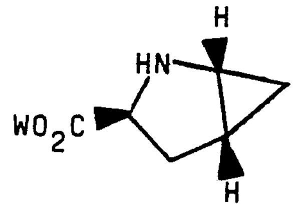

(IIIb) mit Spiegelbild

in denen W eine Carboxy veresternde Gruppe bedeutet, umsetzt und anschließend das Produkt hydriert oder mit einer Säure oder einer Base behandelt, oder

b) daß man zur Herstellung der Verbindungen der Formel I, in der Y und Z zusammen Sauerstoff bedeuten,

$b_1$) eine Verbindung der Formel IV

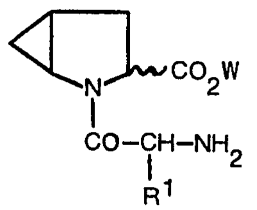

(IV)

in welcher $R^1$ die Bedeutung wie in Formel I und W die Bedeutung wie in den Formeln III a und b besitzen, mit einer Verbindung der Formel V,

$$R^2O_2C—CH=CH—CO—X \quad (V)$$

worin $R^2$ und X die Bedeutungen wie in Formel I haben, umgesetzt und anschließend gegebenenfalls W und/oder $R^2$ unter Bildung der freien Carboxygruppen abgespaltet, oder

$b_2$) eine Verbindung der unter $b_1$) genannten Formel IV mit einer Verbindung der allgemeinen Formel VI, worin $R^2$ die Bedeutung wie in Formel I hat, und mit einer Verbindung der allgemeinen Formel VII,

$$OHC—CO_2R^2 \quad (VI)$$

$$X—CO—CH_3 \quad (VII)$$

worin X die Bedeutung wie in Formel 1 hat, umsetzt und anschließend gegebenenfalls W und/oder $R^2$ unter Bildung der freien Carboxygruppen abspaltet, oder

c) daß man zur Herstellung von Verbindungen der Formel I, in der Y und Z jeweils Wasserstoff bedeuten, eine Verbindung der unter $b_1$) genannten Formel IV mit einer Verbindung der Formel VIII,

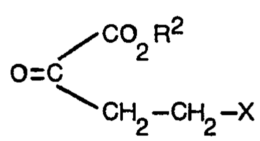

(VIII)

worin $R^2$ und X die Bedeutungen wie in Formel I besitzen, umsetzt, die erhaltenen Schiff-Basen reduziert und anschließend gegebenenfalls W und/oder $R^2$ unter Bildung der freien Carboxygruppen abspaltet, oder

d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, mit einem komplexen Boranat oder Boran-Amin-Komplex reduziert;

die nach a) bis d) erhaltenen Verbindungen der Formel I, in welcher R für Wasserstoff steht, gegebenenfalls in Ester der Formel I, worin R ($C_1$ bis $C_6$)-Alkyl oder ($C_7$ bis $C_9$)-Aralkyl bedeutet, überführt und Verbindungen der Formel I gegebenenfalls in ihre physiologisch verträglichen Salze überführt.

18. Verbindung gemäß einem der Ansprüche 1 bis 16 zur Anwendung als Heilmittel.

19. Mittel, enthaltend eine Verbindung gemäß einem der Ansprüche 1 bis 16.

20. Mittel, enthaltend eine Verbindung gemäß einem der Ansprüche 1 bis 16 in Kombination mit einem Diuretikum.

21. Verbindung gemäß einem der Ansprüche 1 bis 16 in Kombination mit einem Diureticum zur Anwendung als Heilmittel.

22. Verbindung der Formeln IIIa oder IIIb,

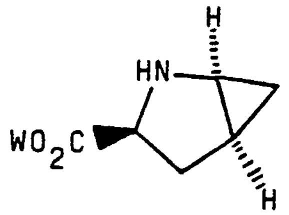

(IIIa) mit Spiegelbild

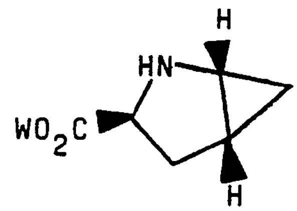

(IIIb) mit Spiegelbild

in welchen W Wasserstoff oder eine Carboxy veresternde Gruppe bedeutet sowie deren Stereoisomerengemisch.

23. Verfahren zur Herstellung von Verbindungen der Formel IIIa oder IIIb gemäß Anspruch 22, dadurch gekennzeichnet, daß man Verbindungen der Formel XI

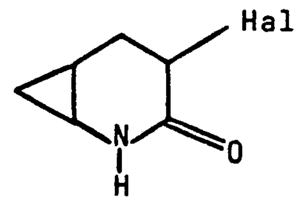

(XI)

in welcher Hal für Halogen steht, in Gegenwart einer Base umlagert und die erhaltenen Verbindungen der Formeln IIIa oder IIIb (W = Wasserstoff) gegebenenfalls verestert.

**Patentansprüche für den Vertragsstaat: AT**

1. Verfahren zur Herstellung von Verbindungen der Formel I,

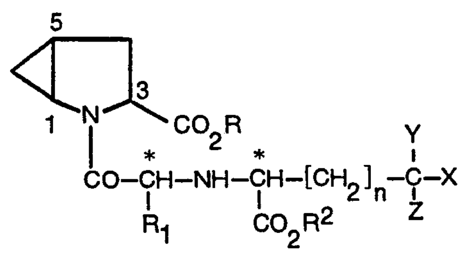

(I)

in der die Wasserstoffatome an den Brückenkopf-C-Atomen zueinander cis-konfiguriert sind und die COOR-Gruppe am C-Atom 3 exo- oder endoständig zum bicyclischen Ringsystem orientiert ist, in der

n = 0 oder 1,

R = Wasserstoff, ($C_1$—$C_6$)-Alkyl, ($C_2$—$C_6$)-Alkenyl oder ($C_6$—$C_{12}$)-Aryl-($C_1$—$C_4$)-Alkyl,

$R^1$ = Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_5$ bis $C_9$)-Cycloalkyl, ($C_5$ bis $C_9$)- Cycloalkenyl, ($C_5$ bis $C_7$)-Cycloalkyl-($C_1$ bis $C_4$)-alkyl, ($C_6$ bis $C_{12}$)-Aryl oder teilhydriertes ($C_6$ bis $C_{12}$)-Aryl, das jeweils durch ($C_1$ bis $C_4$)-Alkyl, ($C_1$ oder $C_2$)-Alkoxy oder Halogen substituiert sein kann, ($C_6$ bis $C_{12}$)-Aryl-($C_1$ bis $C_4$)-alkyl oder ($C_7$ bis $C_{13}$)-Aroyl-($C_1$—$C_2$)alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden α-Aminosäure $R^1$—CH($NH_2$—COOH,

$R^2$ = Wasserstoff, ($C_1$—$C_6$)-Alkyl, ($C_2$—$C_6$)-Alkenyl oder ($C_6$—$C_{12}$)-Aryl-($C_1$—$C_4$)-alkyl,

Y = Wasserstoff oder Hydroxy,

Z = Wasserstoff oder

Y und Z = zusammen Sauerstoff,

X = ($C_1$—$C_6$-Alkyl, ($C_2$—$C_6$)-Alkenyl, ($C_5$-$C_9$)-Cycloalkyl, ($C_6$—$C_{12}$)-Aryl, das durch ($C_1$—$C_4$)-Alkyl, ($C_1$—$C_4$)-Alkoxy, Hydroxy, Halogen, Nitro, Amino, ($C_1$—$C_4$)-Alkylamino, Di-($C_1$—$C_4$)-alkyl-amino und/oder Methylendioxy mono-, di- oder trisubstituiert sein kann,

oder Indol-3-yl bedeuten, sowie deren physiologisch unbedenkliche Salzen, dadurch gekennzeichnet,

a) daß man eine Verbindung der Formel II

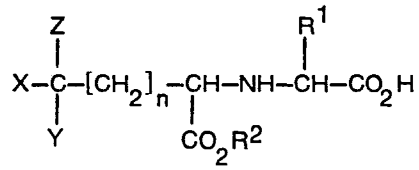

(II)

in der n, X, Y, Z, $R^1$ und $R^2$ die oben definierten Bedeutungen mit Ausnahme von $R^2$ = Wasserstoff haben, mit einer Verbindung der Formeln IIIa oder IIIb oder dem Stereoisomerengemisch

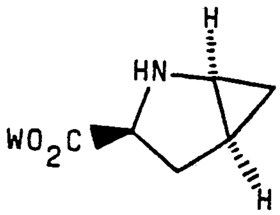

(IIIa) mit Spiegelbild

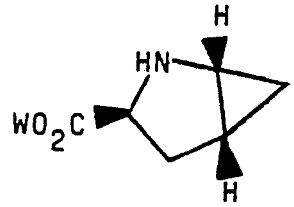

(IIIb) mit Spiegelbild

in denen W eine Carboxy veresternde Gruppe bedeutet, umsetzt und anschließend das Produkt hydriert oder mit einer Säure oder einer Base behandelt, oder

b) daß man zur Herstellung der Verbindungen der Formel I, in der Y und Z zusammen Sauerstoff bedeuten,

$b_1$) eine Verbindung der Formel IV

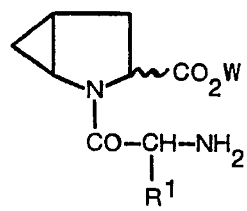

(IV)

in welcher $R^1$ die Bedeutung wie in Formel I und W die Bedeutung wie in den Formeln III a und b besitzen, mit einer Verbindung der Formel V,

$$R^2O_2C—CH=CH—CO—X \quad (V)$$

worin $R^2$ und X die Bedeutungen wie in Formel I haben, umsetzt und anschließend gegebenenfalls W und/oder $R^2$ unter Bildung der freien Carboxygruppen abspaltet, oder

$b_2$) eine Verbindung der unter $b_1$) genannten Formel IV mit einer Verbindung der allgemeinen Formel VI, worin $R^2$ die Bedeutung wie in Formel I hat, und mit einer Verbindung der allgemeinen Formel VII,

$$OHC—CO_2R^2 \quad (VI)$$

$$X—CO—CH_3 \quad (VII)$$

worin X die Bedeutung wie in Formel 1 hat, umsetzt und anschließend gegebenenfalls W und/oder $R^2$ unter Bildung der freien Carboxygruppen abspaltet, oder

c) daß man zur Herstellung von Verbindungen der Formel I, in der Y und Z jeweils Wasserstoff bedeuten, eine Verbindung der unter $b_1$) genannten Formel IV mit einer Verbindung der Formel VIII,

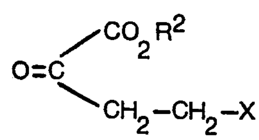

(VIII)

worin $R^2$ und X die Bedeutungen wie in Formel I besitzen, umsetzt, die erhaltenen Schiff-Basen reduziert und anschließend gegebenenfalls W und/oder $R^2$ unter Bildung der freien Carboxygruppen abspaltet, oder

d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, mit einem

komplexen Boranat oder Boran-Amin-Komplex reduziert;
die nach a) bis d) erhaltenen Verbindungen der Formel I, in welcher R für Wasserstoff steht, gegebenenfalls in Ester der Formel I, worin R ($C_1$ bis $C_6$)-Alkyl oder ($C_7$ bis $C_9$)-Aralkyl bedeutet, überführt und Verbindungen der Formel I gegebenenfalls in ihre physiologisch verträglichen Salze überführt.

2. Verfahren gemäß Anspruch 1 zur Herstellung einer Verbindung der Formel I, in welcher das C-Atom in Position 3 des bicyclischen Ringsystems sowie die mit einem Stern markierten C-Atome der Seitenkette jeweils S-Konfiguration aufweisen.

3. Verfahren gemäß einem der Ansprüche 1 oder 2 zur Herstellung einer Verbindung der Formel I, in welcher

n = 1 ist,

$R^1$ = Wasserstoff, Allyl, Vinyl oder eine gegebenenfalls geschützte Seitenkette einer natürlich vorkommenden α-Aminosäure bedeutet und

R, $R^2$, Y, Z und X die in Anspruch 1 definierten Bedeutungen haben.

4. Verfahren gemäß einem der Ansprüche 1 bis 3 zur Herstellung einer Verbindung der Formel I, in welcher

n = 1,

R = Wasserstoff

$R^1$ = Methyl, die gegebenenfalls acylierte Seitenkette von Lysin oder die O-($C_1$—$C_6$)-alkylierte Seitenkette des Tyrosin,

$R^2$ = Wasserstoff, Methyl, Ethyl, Benzyl oder tert. Butyl,

X = Phenyl oder mit Fluor und/oder Chlor mono- oder disubstituiertes Phenyl,

Y = Wasserstoff oder Hydroxy

Z = Wasserstoff oder

Y und Z zusammen Sauerstoff bedeuten.

5. Verfahren gemäß Anspruch 1 zur Herstellung von N-(1-S-Carbethoxy-3-phenyl-propyl)-S-alanyl-cis-2-azabicyclo[3,1,0]hexan-endo-3-S-carbonsäure,

6. Verfahren gemäß Anspruch 1 zur Herstellung von N-(1-S-Carboxy-3-phenyl-propyl-S-alanyl-cis-2-azabicyclo[3,1,0]hexan-endo-3-S-carbonsäure,

7. Verfahren gemäß Anspruch 1 zur Herstellung von N-(1-S-Carbethoxy-3-phenyl-propyl)-S-lysyl-cis-2-azabicyclo[3,1,0]hexan-endo-3-S-carbonsäure,

8. Verfahren gemäß Anspruch 1 zur Herstellung von N-(1-S-Carboxy-3-phenyl-propyl)-S-lysyl-cis-2-azabicyclo[3,1,0]hexan-endo-3-S-carbonsäure,

9. Verfahren gemäß Anspruch 1 zur Herstellung von N-(1-S-Carbethoxy-3-phenyl-propyl)-O-ethyl-S-tyrosyl-cis-2-azabicyclo[3,1,0]hexan-endo-3-S-carbonsäure,

10. Verfahren gemäß Anspruch 1 zur Herstellung von N-(1-S-Carbethoxy-3-phenyl-propyl)-O-methyl-S-tyrosyl-cis-2-azabicyclo[3,1,0]hexan-endo-3-S-carbonsäure,

11. Verfahren gemäß Anspruch 1 zur Herstellung von N-(1-S-Carbethoxy-3-phenyl-propyl)-S-alanyl-cis-2-azabicyclo[3,1,0)hexan-exo-3-S-carbonsäure

12. Verfahren gemäß Anspruch 1 zur Herstellung von N-(1-S-Carboxy-3-phenyl-propyl)-S-alanyl-cis-2-azabicyclo[3,1,0]hexan-exo-3-S-carbonsäure,

13. Verfahren gemäß Anspruch 1 zur Herstellung von N-(1-S-Carbethoxy-3-phenyl-propyl)-S-lysyl-cis-2-azabicyclo[3,1,0]hexan-exo-3-S-carbonsäure,

14. Verfahren gemäß Anspruch 1 zur Herstellung von N-(1-S-Carboxy-3-phenyl-propyl)-S-lysyl-cis-2-azabicyclo[3,1,0]hexan-exo-3-S-carbonsäure,

15. Verfahren gemäß Anspruch 1 zur Herstellung von N-(1-S-Carbethoxy-3-phenyl-propyl)-O-ethyl-S-tyrosyl-cis-2-azabicyclo[3,1,0]hexan-exo-3-S-carbonsäure.

16. Verfahren gemäß Anspruch 1 zur Herstellung von N-(1-S-Carbethoxy-3-phenyl-propyl)-O-methyl-S-tyrosyl-cis-2-azabicyclo[3,1,0]hexan-exo-3-S-carbonsäure,

17. Verfahren gemäß einem der Ansprüche 1 bis 16 zur Herstellung einer Verbindung der Formel I zur Verwendung als Heilmittel,

18. Verfahren zur Herstellung eines Mittels, enthaltend eine Verbindung gemäß einem der Ansprüche 1 bis 16, dadurch gekennzeichnet, daß man diese in eine geeignete Darreichungsform bringt,

19. Verfahren zur Herstellung eines Mittels, enthaltend eine Verbindung gemäß einem der Ansprüche 1 bis 16 in Kombination mit einem Diuretikum, dadurch gekennzeichnet, daß man diese in eine geeignete Darreichungsform bringt,

20. Verfahren zur Herstellung von Verbindungen der Formeln IIIa oder IIIb

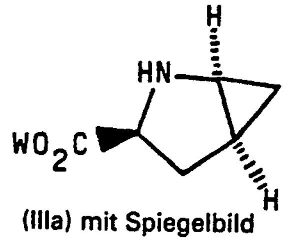

(IIIa) mit Spiegelbild

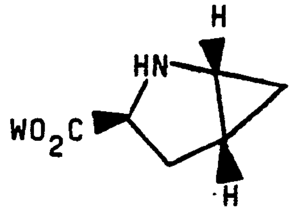

(IIIb) mit Spiegelbild

in welchen W Wasserstoff oder eine Carboxy veresternde Gruppe bedeutet sowie deren Stereoisomerengemisch, dadurch gekennzeichnet, daß man Verbindungen der Formel XI

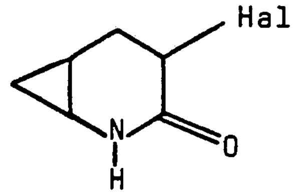

(XI)

in welcher Hal für Halogen steht, in Gegenwart einer Base umlagert und die erhaltenen Verbindungen der Formeln IIIa oder IIIb (W = Wasserstoff) gegebenenfalls verestert.

**Revendications pour les Etats contractants: BE CH DE FR GB IT LI LU NL SE**

1. Composé de formule I

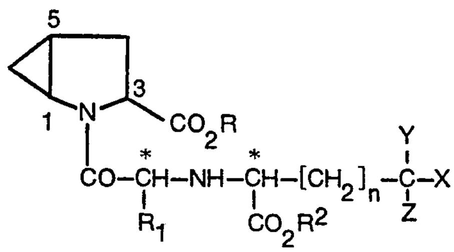

(I)

dans laquelle les atomes d'hydrogène sur les atomes de carbone en tête de pont sont en configuration cis l'un par rapport à l'autre et le groupe COOR sur l'atome de carbone 3 est orienté en exo ou endo par rapport au système bicyclique et dans laquelle

n = 0 ou 1,

R = hydrogène, alkyle en $C_1$—$C_6$, alkényle en $C_1$—$C_6$ ou aryl ($C_6$—$C_{12}$)-alkyle($C_1$—$C_4$),

$R_1$ = hydrogène ou alkyle en $C_1$—$C_6$, éventuellement substitué par amino, acylamino en $C_1$—$C_6$ ou benzoylamino, alkényle en $C_2$—$C_6$, cycloalkyle en $C_5$—$C_9$, cycloalkényle en $C_5$—$C_9$, cycloalkyl($C_5$—$C_7$)-alkyle($C_1$—$C_4$), aryle en $C_6$—$C_{12}$ ou aryle en $C_6$—$C_{12}$ partiellement hydrogéné, pouvant être chaque fois substitué par alkyle en $C_1$ à $C_4$, alcoxy en $C_1$ ou $C_4$ ou halogène, aryl($C_6$—$C_{12}$)-alkyle($C_1$—$C_4$) ou aroyl($C_7$—$C_{13}$)-alkyle($C_1$—$C_2$), qui peuvent tous deux être substitués sur le reste aryle comme défini ci-dessus, un reste hétérocyclique mono- ou bicyclique ayant de 5 à 7 ou de 8 à 10 atomes cycliques, dont 1 à 2 atomes cycliques représentent des atomes de soufre ou d'oxygène et/ou de 1 à 4 atomes cycliques représentent des atomes d'azote, ou bien le groupe latéral, éventuellement protégé, d'un acide α-aminé naturel $R^1$—$CH(NH_2)$-COOH,

$R^2$ est hydrogène, alkyle en $C_1$—$C_6$, alkényle en $C_2$—$C_6$, arylalkyle à aryl($C_6$—$C_{12}$)-alkyle($C_1$—$C_4$),

Y est hydrogène ou hydroxy;

Z est hydrogène, ou

Y et Z ensemble représentent un atome d'oxygène;

X est alkyle en $C_1$—$C_6$, alkényle en $C_2$—$C_6$, cycloalkyle en $C_5$—$C_9$, aryle en $C_6$—$C_{12}$, de préférence phényle, pouvant être mono-, di-ou trisubstitué par alkyle en $C_1$—$C_4$, alcoxy en $C_1$—$C_4$, hydroxy, halogène, nitro, amino, alkylamino en $C_1$—$C_4$, dialkylamino en $C_1$—$C_4$ et/ou méthylènedioxy, ou indol-3-yle, ainsi que leurs sels physiologiquement acceptables.

2. Composé de formule I selon la revendication 1, caractérisé en ce que l'atome de carbone en position 3 du système bicyclique ainsi que les atomes de carbone de la chaîne latérale marqués d'une étoile présentent chaque fois la configuration S.

3. Composé de formule I selon la revendication 1 ou 2, dans laquelle

n = 1,

$R^1$ = hydrogène, allyle, vinyle ou la chaîne latérale, éventuellement protégée, d'un acide α-aminé naturel, et R, $R^2$, Y, Z et X ont les significations données dans la revendication 1.

3. Composé de formule I selon une des revendications 1 à 3, dans laquelle

n = 1,

R = hydrogène,

$R^1$ = méthyle, la chaîne latérale, éventuellement acylée, de la lysine ou la chaîne latérale O-alkylée ($C_1$—$C_6$) de la tyrosine,

$R^2$ = hydrogène, méthyle, éthyle, benzyle ou tert-butyle,

X = phényle ou phényle mono- ou disubstitué par fluor et/ou chlore,

Y = hydrogène ou hydroxy,

Z = hydrogène ou

Y et ensemble représentent l'oxygène.

5. Acide N-(1-S-carbéthoxy-3-phénylpropyl)-S-alanyl-cis-2-azabicyclo[3,1,0]hexane-endo-3-S-carboxylique.

6. Acide N-(1-S-carboxy-3-phénylpropyl)-S-alanyl-cis-2-azabicyclo[3,1,0]hexane-endo-3-S-carboxylique.

7. Acide N-(1-S-carbéthoxy-3-phénylpropyl)-S-lysyl-cis-2-azabicyclo[3,1,0]hexane-endo-3-S-carboxylique.

8. Acide N-(1-S-carboxy-3-phénylpropyl)-S-lysyl-cis-2-azabicyclo[3,1,0]hexane-endo-3-S-carboxylique.

9. Acide N-(1-S-carbéthoxy-3-phénylpropyl)-O-éthyl-S-tyrosyl-cis-2-azabicyclo[3,1,0]hexane-endo-3-S-carboxylique.

10. Acide N-(1-S-carbéthoxy-3-phénylpropyl)-O-méthyl-S-tyrosyl-cis-2-azabicyclo[3,1,0]hexane-endo-3-S-carboxylique.

11. Acide N-(1-S-carbéthoxy-3-phénylpropyl)-S-alanyl-cis-2-azabicyclo[3,1,0]hexane-exo-3-S-carboxylique.

12. Acide N-(1-S-carboxy-3-phénylpropyl)-S-alanyl-cis-2-azabicyclo[3,1,0]hexane-exo-3-S-carboxylique.

13. Acide N-(1-S-carbethoxy-3-phénylpropyl)-S-lysyl-cis-2-azabicyclo[3,1,0]hexane-exo-3-S-carboxylique.

14. Acide N-(1-S-carboxy-3-phénylpropyl)-S-lysyl-cis-2-azabicyclo[3,1,0]hexane-endo-3-S-carboxylique.

15. Acide N-(1-S-carbethoxy-3-phénylpropyl)-O-éthyl-S-tyrosyl-cis-2-azabicyclo[3,1,0]hexane-exo-3-S-carboxylique.

16. Acide N-(1-S-carbethoxy-3-phénylpropyl)-O-méthyl-S-tyrosyl-cis-2-azabicyclo[3,1,0]hexane-exo-3-S-carboxylique.

17. Procédé de préparation de composés selon les revendications 1 à 16, caractérisé en ce que

a) on fait réagir un composé de formule II

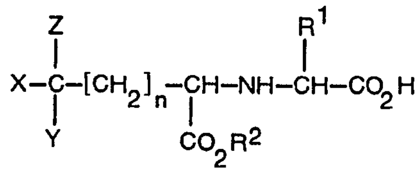

(II)

dans laquelle n, X, Y, Z, $R^1$ et $R^2$ ont les significations données dans la revendication 1, à l'exception de $R_2$ = hydrogène, avec un composé de formules IIIa ou IIIb ou leur mélange stéréoisomère,

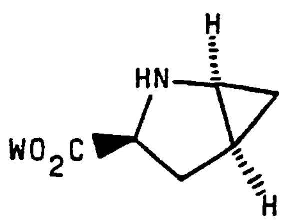

(IIIa) avec image spéculaire

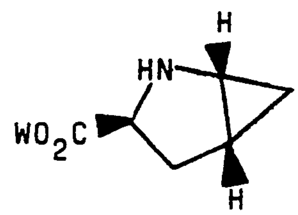

(IIIb) avec image spéculaire

dans lesquelles W est un groupe estérifiant un carboxyle, eet que l'on hydrogène ensuite le produit ou qu'on le traite avec un acide ou une base, ou bien

b) que l'on fait réagir, pour la préparation des composés de formule I, dans laquelle Y et Z ensemble représentent l'oxygène,

$b_1$) un composé de formule IV,

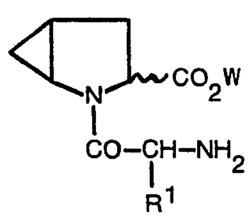

(IV)

dans laquelle $R^1$ a la même signification que dans la formule I et W a la même signification que dans les formules III a et b, avec un composé de formule V,

$$R^2O_2C—CH=CH—CO—X \quad (V)$$

dans laquelle $R^2$ et X ont les mêmes significations que dans la formule I, et que l'on scinde ensuite éventuellement W et/ou

$R^2$, pour former des groupes carboxyliques libres, ou encore

$b_2$) que l'on fait réagir un composé de formule IV mentionnée sous $b_1$) avec un composé de formule générale VI, dans laquelle

$R^2$ a la même signification que dans la formule I, et avec un composé de formule générale VII,

$$OHC—CO_2R^2 \qquad (VI)$$

$$X—CO—CH_3 \qquad (VII)$$

dans laquelle X a la même signification que dans la formule, puis que l'on scinde éventuellement W et/ou $R^2$ former des groupes carboxyliques libres, ou encore

c) que, pur la préparation de composés de formule I dans laquelle Y et Z sont chacun hydrogène, on fait réagir un composé de formule IV mentionnée sous $b_1$) avec un composé de formule VIII,

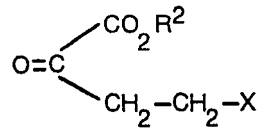
(VIII)

dans laquelle $R^2$ et X ont les mêmes significations que dans la formule I, que l'on réduit les bases de Schiff obtenues et ensuite que l'on scinde éventuellement W et/ou $R^2$ pour former des groupes carboxyliques libres, ou encore

d) que, pour la préparation de composés de formule I, dans laquelle Y = hydroxy et Z = hydrogène, on réduit un composé de formule I, dans laquelle Y et Z ensemble représentent l'oxygène, avec un boranate complexe ou un complexe boraneamine; que l'on transforme éventuellement les composés de formule I obtenus selon les procédés a) à d), dans lesquels R est hydrogène, en leurs esters de formule I, dans laquelle R est alkyle en $C_1$ à $C_6$ ou aralkyle en $C_7$ à $C_9$, et que l'on transforme éventuellement les composés de formule I en leurs sels physiologiquement compatibles.

18. Composé selon l'une des revendications 1 à 16, dans son utilisation come médicament.

19. Agent, contenant un composé selon l'une des revendications 1 à 16.

20. Agent, contenant un composé selon l'une des revendications 1 à 16, en association avec un diurétique.

21. Composé selon l'une des revendications 1 à 16, en association avec un diurétique, pour utilisation come médicament.

22. Composé de formules IIIa ou IIIb (et leurs images spéculaires)

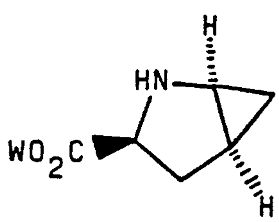
(IIIa) + image spéculaire

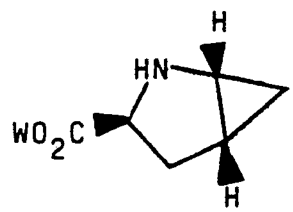
(IIIb) + image spéculaire

dans lesquelles W est hydrogène ou un groupe estérifiant du carboxyle, ainsi que le mélange de ses stéréoisomères.

23. Procéde de préparation de composés de formule IIIa ou IIIb selon a revendication 22, caractérisé en ce que l'on transpose des composés de formule XI,

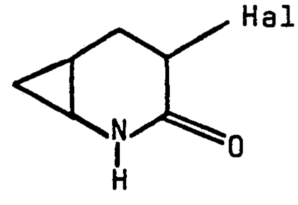
(XI)

dans laquelle Hal représente un atome d'halogène, en présence d'une base, et que l'on estérifie éventuellement les composés obtenus de formules IIIa et IIIb (W = hydrogène).

**Revendications pour l'Etat contractant: AT**

1. Procédé de préparation de composés de formule I

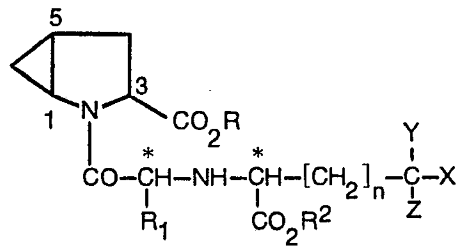
(I)

dans laquelle les atomes d'hydrogène sur les atomes de carbone en tête de pont sont en configuration cis l'un par rapport à l'autre et le groupe COOR sur l'atome de carbone 3 est orienté en exo ou endo par rapport au système bicyclique et dans laquelle

n = 0 ou 1,

R = hydrogène, alkyle en $C_1$—$C_6$, alkényle en $C_1$—$C_6$ ou aryl ($C_6$—$C_{12}$)-alkyle($C_1$—$C_4$),

$R_1$ = hydrogène ou alkyle en $C_1$—$C_6$, éventuellement substitué par amino, acylamino en $C_1$—$C_6$ ou benzoylamino, alkényle en $C_2$—$C_6$, cycloalkyle en $C_5$—$C_9$, cycloalkényle en $C_5$—$C_9$, cycloalkyl($C_5$—$C_7$)-alkyle($C_1$—$C_4$), aryle en $C_6$—$C_{12}$ ou aryle en $C_6$—$C_{12}$ partiellement hydrogéné, pouvant être chaque fois substitué par alkyle en $C_1$ à $C_4$, alcoxy en $C_1$ ou $C_4$ ou halogène, aryl($C_6$—$C_{12}$)-alkyle($C_1$—$C_4$) ou aroyl($C_7$—$C_{13}$)-alkyle($C_1$—$C_2$), qui peuvent tous deux être substitués sur le reste aryle comme défini ci-dessus, un reste hétérocyclique mono- ou bicyclique ayant de 5 à 7 ou de 8 à 10 atomes cycliques, dont 1 à 2 atomes cycliques représentent des atomes de soufre ou d'oxygène et/ou de 1 à 4 atomes cycliques représentent des atomes d'azote, ou bien le groupe latéral, éventuellement protégé, d'un acide α-aminé naturel $R^1$—CH($NH_2$)-COOH,

$R^2$ est hydrogène, alkyle en $C_1$—$C_6$, alkényle en $C_2$—$C_6$, arylalkyle à aryl($C_6$—$C_{12}$)-alkyle($C_1$—$C_4$),

Y est hydrogène ou hydroxy;

Z est hydrogène, ou

Y et Z ensemble représentent un atome d'oxygène;

X est alkyle en $C_1$—$C_6$, alkényle en $C_2$—$C_6$, cycloalkyle en $C_5$—$C_9$, aryle en $C_6$—$C_{12}$, de préférence phényle, pouvant être mono-, di-ou trisubstitué par alkyle en $C_1$—$C_4$, alcoxy en $C_1$—$C_4$, hydroxy, halogène, nitro, amino, alkylamino en $C_1$—$C_4$, dialkylamino en $C_1$—$C_4$ et/ou méthylènedioxy, ou indol-3-yle, ainsi que leurs sels physiologiquement acceptables, caractérisé en ce que

a) on fait réagir un composé de formule II

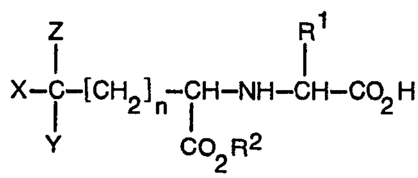
(II)

dans laquelle n, X, Y, Z, $R^1$ et $R^2$ ont les significations données dans la revendication 1, à l'exception de $R_2$ = hydrogène, avec un composé de formules IIIa ou IIIb ou leur mélange stéréoisomère,

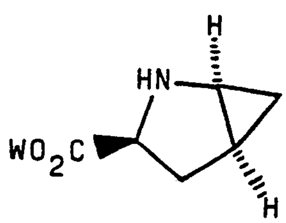
(IIIa) avec image spéculaire

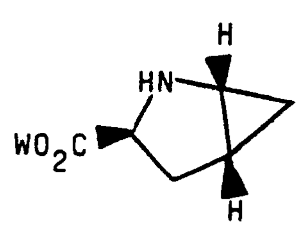
(IIIb) avec image spéculaire

dans lesquelles W est un groupe estérifiant un carboxyle, et que l'on hydrogène ensuite le produit ou qu'on le traite avec un acide ou une base, ou bien

b) que l'on fait réagir, pour la préparation des composés de formule I, dans laquelle Y et Z ensemble représentent l'oxygène,

$b_1$) un composé de formule IV,

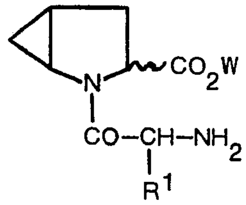

(IV)

dans laquelle $R^1$ a la même signification que dans la formule I et W a la même signification que dans les formules III a et b, avec un composé de formule V,

$$R^2O_2C—CH=CH—CO—X \quad (V)$$

dans laquelle $R^2$ et X ont les mêmes significations que dans la formule I, et que l'on scinde ensuite éventuellement W et/ou

$R^2$, pour former des groupes carboxyliques libres, ou encore

$b_2$) que l'on fait réagir un composé de formule IV mentionnée — sous $b_1$) avec un composé de formule générale VI, dans laquelle

$R^2$ a la même signification que dans la formule I, et avec un composé de formule générale VII,

$$OHC—CO_2R^2 \quad (VI)$$

$$X—CO—CH_3 \quad (VII)$$

dans laquelle X a la même signification que dans la formule I, puis que l'on scinde éventuellement W et/ou $R^2$ former des groupes carboxyliques libres, ou encore

c) que, pour la préparation de composés de formule I dans laquelle Y et Z sont chacun hydrogène, on fait réagir un composé de formule IV mentionnée sous $b_1$) avec un composé de formule VIII,

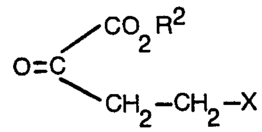

(VIII)

dans laquelle $R^2$ et X ont les mêmes significations que dans la formule I, que l'on réduit les bases de Schiff obtenues et ensuite que l'on scinde éventuellement W et/ou $R^2$ pour former des groupes carboxyliques libres, ou encore

d) que, pour la préparation de composés de formule I, dans laquelle Y = hydroxy ete Z = hydrogène, on réduit un composé de formule I, dans laquelle Y et Z ensemble représentent l'oxygène, avec un boranate complexe ou un complexe boraneamine; que l'on transforme éventuellement les composés de formule I obtenus selon les procédés a) à d), dans lesquels R est hydrogène, en leurs esters de formule I, dans laquelle R est alkyle en $C_1$ à $C_6$ ou aralkyle en $C_7$ à $C_9$, et que l'on transforme éventuellement les composés de formule I en leurs sels physiologiquement compatibles.

2. Procédé de préparation, selon la revendication 1, d'un composé de formule I, dans laquelle l'atome de carbone en position 3 du système bicyclique ainsi que les atomes de carbone de la chaîne latérale marqués d'une étoile présentent chaque fois la configuration S.

3. Procédé de préparation, selon la revendication 1 ou 2, d'un composé de formule I, dans laquelle

n = 1,

$R^1$ = hydrogène, allyle, vinyle ou la chaîne latérale, éventuellement protégée, d'un acide α-aminé naturel, et R, $R^2$, Y, Z et X ont les significations données dans la revendication 1.

3. Procédé de préparation, selon une des revendications 1 à 3, d'un composé de formule I, dans laquelle

n = 1,

R = hydrogène,

$R^1$ = méthyle, la chaîne latérale, éventuellement acylée de la lysine ou la chaîne latérale O-alkylée ($C_1$—$C_6$) de la tyrosine,

$R^2$ = hydrogène, méthyle, éthyle, benzyle ou tert-butyle,

X = phényle ou phényle mono- ou disubstitué par fluor et/ou chlore,

Y = hydrogène ou hydroxy,

Z = hydrogène ou

Y et Z ensemble représentent l'oxygène.

5. Procédé de préparation, selon la revendication 1, de l'acide N-(1-S-carbéthoxy-3-phénylpropyl)-S-alanyl-cis-2-azabicyclo[3,1,0]hexane-endo-3-S-carboxylique.

6. Procédé de préparation, selon la revendication 1, de l'acide N-(1-S-carboxy-3-phénylpropyl)-S-alanyl-cis-2-azabicyclo[3,1,0]hexane-endo-3-S-carboxylique.

7. Procédé de préparation, selon la revendication 1, de l'acide N-(1-S-carbéthoxy-3-phénylpropyl)-S-lysyl-cis-2-azabicyclo[3,1,0]hexane-endo-3-S-carboxylique.

8. Procédé de préparation, selon la revendication 1, de l'acide N-(1-S-carboxy-3-phénylpropyl)-S-lysyl-cis-2-azabicyclo[3,1,0]hexane-endo-3-S-carboxylique.

9. Procédé de préparation, selon la revendication 1, de l'acide N-(1-S-carbéthoxy-3-phénylpropyl)-O-éthyl-S-tyrosyl-cis-2-azabicyclo[3,1,0]hexane-endo-3-S-carboxylique.

10. Procédé de préparation, selon la revendication 1, de l'acide N-(1-S-carbéthoxy-3-phénylpropyl)-O-méthyl-S-tyrosyl-cis-2-azabicyclo[3,1,0]hexane-endo-3-S-carboxylique.

11. Procédé de préparation, selon la revendication 1, de l'acide N-(1-S-carbéthoxy-3-phénylpropyl)-S-alanyl-cis-2-azabicyclo[3,1,0]hexane-exo-3-S-carboxylique.

12. Procédé de préparation, selon la revendication 1, de l'acide N-(1-S-carboxy-3-phénylpropyl)-S-alanyl-cis-2-azabicyclo[3,1,0]hexane-exo-3-S-carboxylique.

13. Procédé de préparation, selon la revendication 1, de l'acide N-(1-S-carbéthoxy-3-phénylpropyl)-S-lysyl-cis-2-azabicyclo[3,1,0]hexane-exo-3-S-carboxylique.

14. Procédé de préparation, selon la revendication 1, de l'acide N-(1-S-carboxy-3-phénylpropyl)-S-lysyl-cis-2-azabicyclo[3,1,0]hexane-endo-3-S-carboxylique.

15. Procédé de préparation, selon la revendication 1, de l'acide N-(1-S-carbéthoxy-3-phénylpropyl)-O-éthy-S-tyrosyl-cis-2-azabicyclo[3,1,0]hexane-exo-3-S-carboxylique.

16. Procédé de préparation, selon la revendication 1, de l'acide N-(1-S-carbéthoxy-3-phénylpropyl)-O-méthyl-S-tyrosyl-cis-2-azabicyclo[3,1,0]hexane-exo-3-S-carboxylique.

17. Procédé de préparation, selon l'une des revendications 1 à 16, d'un composé de formule I utilisé come médicament.

18. Procédé de fabrication d'un agent, contenant un composé selon l'une des revendications 1 à 16, caractérisé en ce qu'on le met sous une forme d'administration appropriée.

19. Procédé de fabrication d'un agent, contenant un composé selon l'une des revendications 1 à 16 en association avec un diurétique, caractérisé en ce qu'on le met sous une forme d'administration appropriée.

20. Procédé de préparation de composés de formule IIIa ou IIIb (et leurs images spéculaires),

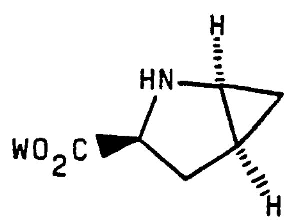

(IIIa) + image spéculaire

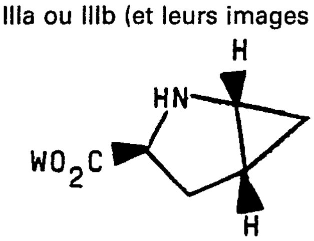

(IIIb) + image spéculaire

dans lesquelles W est hydrogène ou un groupe estérifiant du carboxyle, ainsi que le mélange de leurs stéréoisomères, caractérisé en ce que l'on transpose des composés de formule XI,

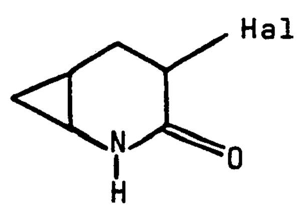

(XI)

dans laquelle Hal représente un atome d'halogène, en présence d'une base, et que l'on estérifie éventuellement les composés obtenus de formules IIIa et IIIb (W = hydrogène).

**Claims for the Contracting States: BE CH DE FR GB IT LI LU NL SE**

1. A compound of the formula I

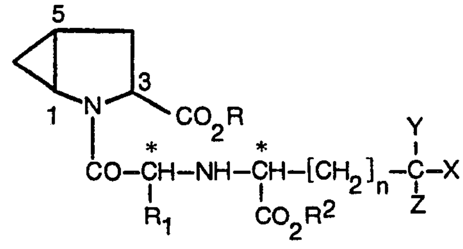

(I)

in which the hydrogen atoms at the bridgehead carbon atoms have the cis configuration with respect to one another, and the COOR group on carbon atom 3 is oriented exo or endo to the bicyclic ring system, and in which

n denotes 0 or 1,

R dentoes hydrogen, $(C_1—C_6)$-alkyl, $(C_2—C_6)$-alklenyl or $(C_6—C_{12})$-aryl-$(C_1—C_4)$-alkyl,

$R_1$ denotes hydrogen or ($C_1$ to $C_6$)-alkyl, which can optionally be substituted by amino, ($C_1$ to $C_6$)-acylamino or benzoylamino, ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to $C_4$)-alkyl, ($C_6$ to $C_{12}$)-aryl or partially hydrogenated ($C_6$ to $C_{12}$)-aryl, each of which can be substituted by ($C_1$ to $C_4$)-alkyl, ($C_1$ or $C_2$)-alkoxy or halogen, ($C_6$ to $C_{12}$)-aryl-($C_1$ to $C_4$)-alkyl or ($C_7$ to $C_{13}$)-aroyl-$(C_1—C_2)$-alkyl, both of which can be substituted in the aryl radical as defined above, a monocyclic or bicyclic heterocyclic radical having 5 to 7 or 8 to 10 ring atoms respectively, 1 or 2 of these ring atoms being sulfur or oxygen atoms and/or 1 to 4 of these ring atoms being nitrogen atoms, or an optionally protected side chain of a naturally occurring α-amino acid $R^1—CH(NH_2)—COOH$,

$R^2$ denotes hydrogen, $(C_1—C_6)$-alkyl, $(C_2—C_6)$-alkenyl or $(C_6—C_{12})$-aryl-$(C_1—C_4)$-alkyl,

Y denotes hydrogen or hydroxyl,

Z denotes hydrogen, or

Y and Z together denote oxygen, and

X denotes $(C_1—C_6)$-alkyl, $(C_2—C_6)$-alkenyl, $(C_5—C_9)$-cycloalkyl, $(C_6—C_{12})$-aryl, which can be monosubstituted, disubstituted or trisubstituted by $(C_1—C_4)$-alkyl, $(C_1—C_4)$-alkoxy, hydroxyl, halogen, nitro, amino, $(C_1—C_4)$-alkylamino, di-$(C_1—C_4)$-alkylamino and/or methylenedioxy, or 3-indolyl, and its physiologically acceptable salts.

2. A compound of the formula I as claimed in claim 1, wherein the carbon atom in position 3 of the bicyclic ring system and the carbon atoms in the side chain labeled with an asterisk each have the S configuration.

3. A compound of the formula I as claimed in one of claims 1 or 2, in which

n is 1,

$R^1$ denotes hydrogen, allyl, vinyl or an optionally protected side chain of a naturally occurring α-amino acid, and

R, $R^2$, Y, Z and X have the meanings defined in claim 1.

4. A compound of the formula I according to one of claims 1 to 3, in which

n denotes 1,

R denotes hydrogen,

$R^1$ denotes methyl, the optionally acylated side chain of lysine or the O-$(C_1—C_6)$-alkylated side chain of tyrosine,

$R^2$ denotes hydrogen, methyl, ethyl, benzyl or tert.-butyl,

X denotes phenyl or phenyl which is monosubstituted or disubstituted by fluorine and/or chlorine,

Y denotes hydrogen or hydroxyl,

Z denotes hydrogen, or

Y and Z together denote oxygen.

5. N-(1-S-carboethoxy-3-phenylpropyl)-S-alanyl-cis-2-azabicyclo[3,1,0]hexane-endo-3-S-carboxylic acid.

6. N-(1-S-carboxy-3-phenylpropyl)-S-alanyl-cis-2-azabicyclo[3,1,0]hexane-endo-3-S-carboxylic acid.

7. N-(1-S-carboethoxy-3-phenylpropyl)-S-lysyl-cis-2-azabicyclo[3,1,0]hexane-endo-3-S-carboxylic acid.

8. N-(1-S-carboxy-3-phenylpropyl)-S-lysyl-cis-2-azabicyclo[3,1,0]hexane-endo-3-S-carboxylic acid.

9. N-(1-S-carboethoxy-3-phenylpropyl)-O-ethyl-S-tyrosyl-cis-2-azabicyclo[3,1,0]hexane-endo-3-S-carboxylic acid.

10. N-(1-S-carboethoxy-3-phenylpropyl)-O-methyl-S-tyrosyl-cis-2-azabicyclo[3,1,0]hexane-endo-3-S-carboxylic acid.

11. N-(1-S-carboethoxy-3-phenylpropyl)-S-alanyl-cis-2-azabicyclo[3,1,0]hexane-exo-3-S-carboxylic acid.

12. N-(1-S-carboxy-3-phenylpropyl)-S-alanyl-cis-2-azabicyclo[3,1,0]hexane-exo-3-S-carboxylic acid.

13. N-(1-S-carboethoxy-3-phenylpropyl)-S-lysyl-cis-2-azabicyclo[3,1,0]hexane-endo-3-S-carboxylic acid.

14. N-(1-S-carboxy-3-phenylpropyl)-S-lysyl-cis-2-azabicyclo[3,1,0]hexane-exo-3-S-carboxylic acid.

15. N-(1-S-carboethoxy-3-phenylpropyl)-O-ethy-S-tyrosyl-cis-2-azabicyclo[3,1,0]hexane-exo-3-S-carboxylic acid.

16. N-(1-S-carboethoxy-3-phenylpropyl)-O-methyl-S-tyrosyl-cis-2-azabicyclo[3,1,0]hexane-exo-3-S-carboxylic acid.

17. A process for the preparation of a compound as claimed in claims 1 to 16, which comprises

a) reacting a compound of the formula II

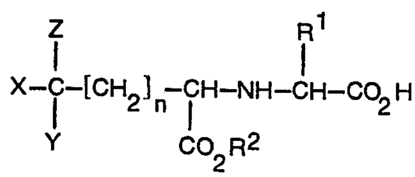

(II)

in which n, X, Y, Z, $R^1$ and $R^2$ have the meanings defined in claim 1, with the exception of $R^2$ = hydrogen, with a compound of the formulae IIIa or IIIb or the mixture of stereoisomers

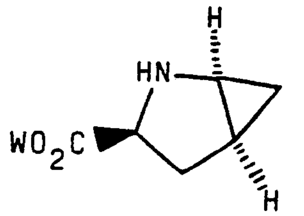

(IIIa) with mirror image

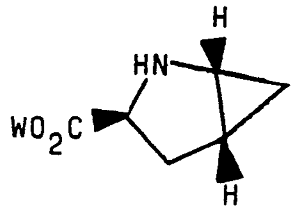

(IIIb) with mirror image

in which W denotes a group esterifying carboxyl, and then hydrogenating the product or treating it with an acid or a base, or

b) for the preparation of a compound of the formula I in which Y and Z together denote oxygen,

$b_1$) reacting a compound of the formula IV

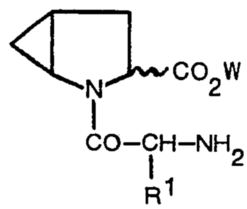

(IV)

in which $R^1$ has the meaning as in formula I and W has the meaning as in the formulae IIIa and b, with a compound of the formula V

$$R^2O_2C—CH=CH—CO—X \qquad (V)$$

in which $R^2$ and X have the meanings as in formula I, and then, where appropriate, splitting off W and/or $R^2$ with the formation of the free carboxyl groups, or

$b_2$) reacting a compound of the formula IV mentioned under

$b_1$) with a compound of the general formula VI, in which $R^2$ has the meaning as in formula I, and with a compound of the general formula VII,

$$OHC—CO_2R^2 \qquad (VI)$$

$$X—CO—CH_3 \qquad (VII)$$

in which X has the meaning as in formula I, and then, where appropriate, splitting off W and/or $R^2$ with the formation of the free carboxyl groups, or

c) for the preparation of a compound of the formula I, in which Y and Z each denote hydrogen, reacting a compound of the formula IV mentioned under $b_1$) with a compound of the formula VIII,

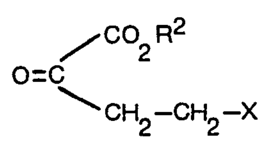

(VIII)

in which $R^2$ and X have the meanings as in formula I, reducing the resulting Schiff's bases and then, where appropriate, splitting off W and/or $R^2$ with the formation of the free carboxyl groups, or

d) for the preparation of a compound of the formula I, in which Y denotes hydroxyl and Z denotes hydrogen, reducing a compound of the formula I, in which Y and Z together denote oxygen, with a complex boranate or borane-amine complex; converting, where appropriate, a compound of the formula I in which R represents hydrogen, which has been obtained according to a) to d), into an ester of the formula I in which R denotes ($C_1$ to $C_6$)-alkyl or ($C_7$ to $C_9$)-aralkyl, and, where appropriate, converting a compound of the formula I into its physiologically tolerated salts.

18. A compound as claimed in one of claims 1 to 16, for use as a medicament.

19. An agent containing a compound as claimed in one of claims 1 to 16.

20. An agent containing a compound as claimed in one of claims 1 to 16 combined with a diuretic.

21. A compound as claimed in one of claims 1 to 16, combined with a diuretic, for use as a medicament.

22. A compound of the formulae IIIa or IIIb,

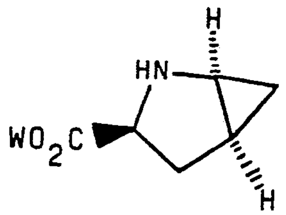

(IIIa) + mirror image

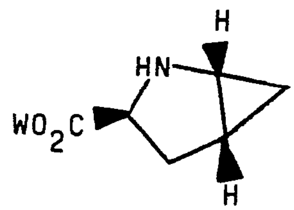

(IIIb) + mirror image

in which W denotes hydrogen or a group esterifying carboxyl, and the mixture of its stereoisomers.

23. A process for the preparation of compounds of the formula IIIa or IIIb as claimed in claim 22, which comprises rearranging compounds of the formula XI

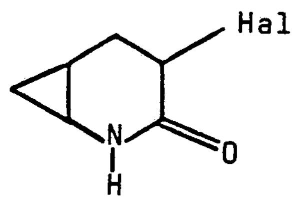

(XI)

in which Hal represents halogen, in the presence of a base, and, where appropriate, esterifying the resulting compounds of the formulae IIIa or IIIb (W = hydrogen).

**Claims for the Contracting State: AT**

1. A process for the preparation of compounds of the formula I

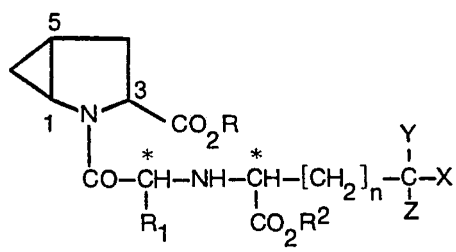

(I)

in which the hydrogen atoms at the bridgehead carbon atoms have the cis configuration with respect to one another, and the COOR group on carbon atom 3 is oriented exo or endo to the bicyclic ring system, and in which

n denotes 0 or 1,

R denotes hydrogen, ($C_1$—$C_6$)-alkyl, ($C_2$—$C_6$)-alklenyl or ($C_6$—$C_{12}$)-aryl-($C_1$—$C_4$)-alkyl,

$R_1$ denotes hydrogen or ($C_1$ to $C_6$)-alkyl, which can optionally be substituted by amino, ($C_1$ to $C_6$)-acylamino or benzoylamino, ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to $C_4$)-alkyl, ($C_6$ to $C_{12}$)-aryl or partially hydrogenated ($C_6$ to $C_{12}$)-aryl, each of which can be substituted by ($C_1$ to $C_4$)-alkyl, ($C_1$ or $c_2$)-alkoxy or halogen, ($C_6$ to $C_{12}$)-aryl-($C_1$ to $C_4$)-alkyl or ($C_7$ to $C_{13}$)-aroyl-($C_1$—$C_2$)-alkyl, both of which can be substituted in the aryl radical as defined above, a monocyclic or bicyclic heterocyclic radical having 5 to 7 or 8 to 10 ring atoms respectively, 1 or 2 of these ring atoms being sulfur or oxygen atoms and/or 1 to 4 of these ring atoms being nitrogen atoms, or an optionally protected side chain of a naturally occurring α-amino acid $R^1$—$CH(NH_2)$—COOH,

$R^2$ denotes hydrogen, ($C_1$—$C_6$)-alkyl, ($C_2$—$C_6$)-alkenyl or ($C_6$—$C_{12}$)-aryl-($C_1$—$C_4$)-alkyl,

Y denotes hydrogen or hydroxyl,

Z denotes hydrogen, or

Y and Z together denote oxygen, and

X denotes ($C_1$—$C_6$)-alkyl, ($C_2$—$C_6$)-alkenyl, ($C_5$—$C_9$)-cycloalkyl, ($C_6$—$C_{12}$)-aryl, which can be monosubstituted, disubstituted or trisubstituted by ($C_1$—$C_4$)-alkyl, ($C_1$—$C_4$)-alkoxy, hydroxyl, halogen, nitro, amino, ($C_1$—$C_4$)-alkylamino, di-($C_1$—$C_4$)-alkylamino and/or methylenedioxy, or 3-indolyl, and their physiologically acceptable salts which comprises,

a) reacting a compound of the formula II

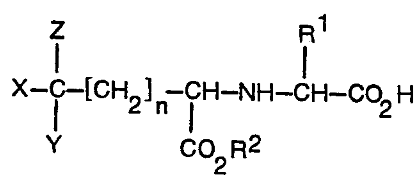

(II)

in which n, X, Y, Z, $R^1$ and $R^2$ have the meanings defined above, with the exception of $R^2$ = hydrogen, with a compound of the formulae IIIa or IIIb or the mixture of stereoisomers

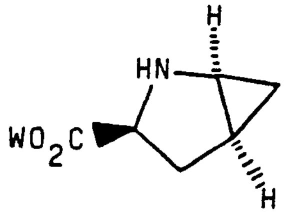

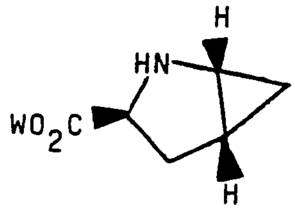

(IIIa) with mirror image (IIIb) with mirror image

in which W denotes a group esterifying carboxyl, and then hydrogenating the product or treating it with an acid or a base, or

b) for the preparation of a compound of the formula I in which Y and Z together denote oxygen,

$b_1$) reacting a compound of the formula IV

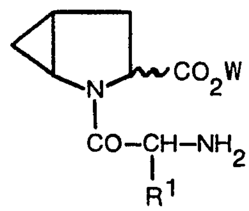

(IV)

in which $R^1$ has the meaning as in formula I and W has the meaning as in the formulae IIIa and b, with a compound of the formula V

$$R^2O_2C—CH=CH—CO—X \quad (V)$$

in which $R^2$ and X have the meanings as in formula I, and then, where appropriate, splitting off W and/or $R^2$ with the formation of the free carboxyl groups, or

$b_2$) reacting a compound of the formula IV mentioned under

$b_1$) with a compound of the general formula VI, in which $R^2$ has the meaning as in formula I, and with a compound of the general formula VII,

$$OHC—CO_2R^2 \quad (VI)$$

$$X—CO—CH_3 \quad (VII)$$

in which X has the meaning as in formula I, and then, where appropriate, splitting off W and/or $R^2$ with the formation of the free carboxyl groups, or

c) for the preparation of compounds of the formula I, in which Y and Z each denote hydrogen, reacting a compound of the formula IV mentioned under $b_1$) with a compound of the formula VIII,

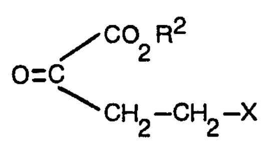

(VIII)

in which $R^2$ and X have the meanings as in formula I, reducing the resulting Schiff's bases and then, where appropriate, splitting off W and/or $R^2$ with the formation of the free carboxyl groups, or

d) for the preparation of a compound of the formula I, in which Y denotes hydroxyl and Z denotes hydrogen, reducing a compound of the formula I, in which Y and Z together denote oxygen, with a complex boranate or borane-amine complex; converting, where appropriate, a compound of the formula I in which R represents hydrogen, which have been obtained according to a) to d), into an ester of the formula I in which R denotes ($C_1$ to $C_6$)-alkyl or ($C_7$ to $C_9$)-aralkyl, and, where appropriate, converting a compound of the formula I into its physiologically tolerated salts.

2. The process as claimed in claim 1 for the preparation of a compound of the formula I, wherein the carbon atom in position 3 of the bicyclic ring system and the carbon atoms in the side chain labeled with an asterisk each have the S configuration.

3. The process as claimed in one of claims 1 or 2 for the preparation of a compound of the formula I, wherein

n is 1,
$R^1$ denotes hydrogen, allyl, vinyl or an optionally protected side chain of a naturally occurring α-amino acid, and
R, $R^2$, Y, Z and X have the meanings defined in claim 1.

4. The process as claimed in one of claims 1 to 3 for the preparation of a compound of the formula I
n denotes 1,
R denotes hydrogen,
$R^1$ denotes methyl, the optionally acylated side chain of lysine or the O-($C_1$—$C_6$)-alkylated side chain of tyrosine,
$R^2$ denotes hydrogen, methyl, ethyl, benzyl or tert-butyl,
X denotes phenyl or phenyl which is monosubstituted or disubstituted by fluorine and/or chlorine,
Y denotes hydrogen or hydroxyl,
Z denotes hydrogen, or
Y and Z together denote oxygen.

5. The process as claimed in claim 1 for the preparation of N-(1-S-carboethoxy-3-phenylpropyl)-S-alanyl-cis-2-azabicyclo[3,1,0]hexane-endo-3-S-carboxylic acid.

6. The process as claimed in claim 1 for the preparation of N-(1-S-carboxy-3-phenylpropyl)-S-alanyl-cis-2-azabicyclo[3,1,0]hexane-endo-3-S-carboxylic acid.

7. The process as claimed in claim 1 for the preparation of N-(1-S-carboethoxy-3-phenylpropyl)-S-lysyl-cis-2-azabicyclo[3,1,0]hexane-endo-3-S-carboxylic acid.

8. The process as claimed in claim 1 for the preparation of N-(1-S-carboxy-3-phenylpropyl)-S-lysyl-cis-2-azabicyclo[3,1,0]hexane-endo-3-S-carboxylic acid.

9. The process as claimed in claim 1 for the preparation of N-(1-S-carbethoxy-3-phenylpropyl)-O-ethyl-S-tyrosyl-cis-2-azabicyclo[3,1,0]hexane-endo-3-S-carboxylic acid.

10. The process as claimed in claim 1 for the preparation of N-(1-S-carboethoxy-3-phenylpropyl)-O-methyl-S-tyrosyl-cis-2-azabicyclo[3,1,0]hexane-endo-3-S-carboxylic acid.

11. The process as claimed in claim 1 for the preparation of N-(1-S-carboethoxy-3-phenylpropyl)-S-alanyl-cis-2-azabicyclo[3,1,0]hexane-exo-3-S-carboxylic acid.

12. The process as claimed in claim 1 for the preparation of N-(1-S-carboxy-3-phenylpropyl)-S-alanyl-cis-2-azabicyclo[3,1,0]hexane-exo-3-S-carboxylic acid.

13. The process as claimed in claim 1 for the preparation of N-(1-S-carboethoxy-3-phenylpropyl)-S-lysyl-cis-2-azabicyclo[3,1,0]hexane-exo-3-S-carboxylic acid.

14. The process as claimed in claim 1 for the preparation of N-(1-S-carboxy-3-phenylpropyl)-S-lysyl-cis-2-azabicyclo[3,1,0]hexane-endo-3-S-carboxylic acid.

15. The process as claimed in claim 1 for the preparation of N-(1-S-carboethoxy-3-phenylpropyl)-O-ethy-S-tyrosyl-cis-2-azabicyclo[3,1,0]hexane-exo-3-S-carboxylic acid.

16. The process as claimed in claim 1 for the preparation of N-(1-S-carboethoxy-3-phenylpropyl)-O-methyl-S-tyrosyl-cis-2-azabicyclo[3,1,0]hexane-exo-3-S-carboxylic acid.

17. The process as claimed in one of claims 1 to 16 for the preparation of a compound of the formula I for use as a medicament.

18. A process for the preparation of an agent containing a compound as claimed in one of claims 1 to 16, which comprises converting the compound into a form suitable for administration.

19. A process for the preparation of an agent containing a compound as claimed in one of claims 1 to 16 combinied with a diuretic, which comprises converting the compound into a form suitable for administration.

20. A process for the preparation of compounds of the formulae IIIa or IIIb

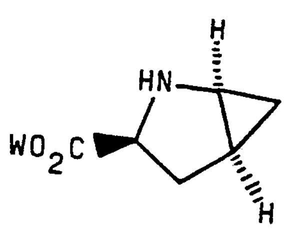

(IIIa) + mirror image

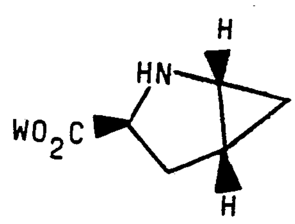

(IIIb) + mirror image

in which W denotes hydrogen or a group esterifying carboxyl, and the mixture of their stereoisomers, which comprises rearranging compounds of the formula XI

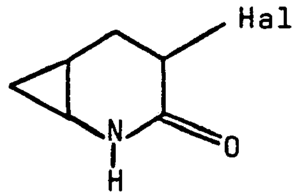

(XI)

in which Hal represents halogen, in the presence of a base, and, where appropriate, esterifying the resulting compounds of the formulae IIIa or IIIb (W = hydrogen).